(19) Europäisches Patentamt
European Patent Office
Office européen des brevets

(11) **EP 4 063 481 A1**

(12) **EUROPEAN PATENT APPLICATION**

(43) Date of publication:
**28.09.2022 Bulletin 2022/39**

(21) Application number: **22156878.5**

(22) Date of filing: **30.07.2015**

(51) International Patent Classification (IPC):
**C12M 1/42** (2006.01)     **G01N 33/483** (2006.01)
**G01N 27/447** (2006.01)

(52) Cooperative Patent Classification (CPC):
**G01N 27/44791; B01L 3/502715; B01L 3/502761;
G01N 27/44704; G01N 33/49; G01N 33/721;
G01N 33/726; B01L 2200/0652; B01L 2300/0636;
B01L 2300/0816; B01L 2400/0487; G01N 2800/22**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.07.2014 US 201462030964 P**

(62) Document number(s) of the earlier application(s) in
accordance with Art. 76 EPC:
**15827038.9 / 3 186 356**

(71) Applicant: **Case Western Reserve University
Cleveland, OH 44106 (US)**

(72) Inventors:
• **GURKAN, Umut, A.**
**Cleveland, OH 44106 (US)**
• **ALAPAN, Yunus**
**Cleveland Heights, OH 44106 (US)**

• **LITTLE, Jane**
**Cleveland Heights, OH 44106 (US)**
• **PICCONE, Connie**
**Cleveland, OH 44106 (US)**
• **UNG, Ryan Dang-Quan**
**Irvine, CA 92603 (US)**

(74) Representative: **Maiwald Patent- und
Rechtsanwaltsgesellschaft mbH
Engineering
Elisenhof
Elisenstrasse 3
80335 München (DE)**

Remarks:
This application was filed on 15-02-2022 as a
divisional application to the application mentioned
under INID code 62.

(54) **BIOCHIPS TO DIAGNOSE HEMOGLOBIN DISORDERS AND MONITOR BLOOD CELLS**

(57)     A biochip compatible with very small blood sample volumes is used for detecting for detecting hemoglobin disorders and monitoring disorders associated with aberrant blood cell deformability and adhesion, including disease severity, upcoming pain crisis, treatment response, and treatment effectiveness in a clinically meaningful way.

Figs. 1A-E

**Description**

**RELATED APPLICATION**

This application claims priority from U.S. Provisional Application No. 62/030,964, filed July 30, 2014, the subject matter of which is incorporated herein by reference in its entirety.

**FIELD OF THE INVENTION**

The present invention is related to biochips, and particularly relates to biochips that rapidly and easily diagnose or identify hemoglobin disorders, such as hemoglobinopathies (*e.g.,* sickle cell disease (SCD)) as well as biochips that monitor patient health and disease progression based on blood cell analysis.

**BACKGROUND**

About 3 million people worldwide suffer from sickle cell disease (SCD), mostly in Africa, India and the Middle East, with an estimated 100,000 affected in the U.S., according to the Centers for Disease Control and Prevention. SCD affects 1 in 375 African American newborns born in the U.S. "U.S. Preventive Services Task Force. Screening for Hemoglobinopathies." In: Guide to Clinical Preventive Services, 2nd edition. 2nd edition ed: International Medical Publishing; 485-494 (1996).

The World Health Organization (WHO) has declared SCD as a public health priority [1-3]. The greatest burden of SCD is in low-income countries, especially in Africa. Estimated 50-80% of the babies born with SCD in Africa die before the age of 5 (*i.e.,* more than 600 babies die every day) due to lack of diagnosis [2, 4]. Very few infants are screened in Africa because of the high cost and level of skill needed to run traditional tests [5]. Current methods are too costly and take too much time (2-6 weeks) to enable equitable and timely diagnosis to save lives [5]. It is estimated by the WHO that, 70% of SCD related deaths are preventable with simple, cost-efficient interventions, such as early point-of-care (POC) diagnosis by newborn screening followed by treatment and care [6]. The diagnostic barrier can be broken with low-cost, POC tools that facilitate early detection immediately after birth [5].

More than 800 children are born with SCD every day in Africa, and more than half of them die in childhood due to lack of diagnosis and early treatment. Modell et al., "Global epidemiology of haemoglobin disorders and derived service indicators." Bulletin of the World Health Organization 86:480-487 (2008); Makani et al., "Mortality in sickle cell anemia in Africa: a prospective cohort study in Tanzania" PloS one 6:e14699 (2011); and McGann et al., "A prospective newborn screening and treatment program for sickle cell anemia in Luanda, Angola. American journal of hematology 88:984-989 (2013). In all 50 states of America, and the District of Columbia, hemoglobin screening of newborns is mandated in order to diagnose SCD early, so that, monitoring and treatment can be started immediately. In: National Institutes of Health. The Management of Sickle Cell Disease 4th Ed., NIH Publication No. 02-2117 NIH National Heart, Lung and Blood Institute (2002).

Early diagnosis through newborn screening, followed by simple interventions, has dramatically reduced the SCD related mortality in the US. However, these strategies have not been widely available in Africa and other third world countries, due to limited resources. What is needed is a point-of-care biochip-based hemoglobin screening method.

**SUMMARY**

Embodiments described herein relate to an electrophoresis biochip and system for detecting hemoglobin disorders, such as sickle cell disease (SCD). The biochip includes a housing, first and second buffer ports, a sample loading port, and first and second electrodes. The housing also includes a microchannel that extends from a first end to a second end of the housing. The microchannel contains cellulose acetate paper that is at least partially saturated with an alkaline buffer solution. The first buffer port and the second buffer extend, respectively, through the first end and second of the housing to the microchannel and cellulose acetate paper. The first buffer port and the second buffer port are capable of receiving the alkaline buffer solution that at least partially saturates the cellulose acetate paper. The sample loading port can receive a blood sample and extends through the first end of the housing to the microchannel and cellulose acetate paper. The first electrode and the second electrode can generate an electric field across the cellulose acetate paper effective to promote migration of hemoglobin variants in the blood sample along the cellulose acetate paper. The first electrode and second electrode can extend, respectively, through the first buffer port and the second port to the cellulose acetate paper.

In some embodiments, the housing can include a viewing area for visualizing the cellulose acetate paper and hemoglobin variant migration. The electrophoresis biochip can further include an imaging system for visualizing and quantifying hemoglobin variant migration along the cellulose acetate paper for blood samples introduced into the sample loading port.

The first electrode and the second electrode can be connected to a power supply. The power supply can generate an electric field of about 1V to about 400V. In some embodiments, the voltage applied to the biochip by the electrodes does not exceed 250V.

In other embodiments, the blood sample introduced into the sample loading port can be less than about 10 microliters. In some embodiments, the buffer solution can include alkaline tris/Borate/EDTA buffer solution. The first electrode and the second electrode can include graphite or carbon electrodes.

In some embodiments, the housing can include a top cap, bottom cap, and a channel spacer interposed between the top cap and the bottom cap. The channel spacer can define the channel in the housing. The top cap, bottom cap, and channel spacer can be formed from at least one of glass or plastic.

In other embodiments, the imaging system can include a mobile phone imaging system to visualize and quantify hemoglobin variant migration. The mobile phone imaging system can include a mobile telephone that is used to image hemoglobin variant migration and a software application that recognizes and quantifies the hemoglobin band types and thicknesses to make a diagnostic decision. In some embodiments, the hemoglobin band types can include hemoglobin types C/A, S, F, and A0.

In other embodiments, the electrophoresis biochip can be used to diagnose whether the subject has hemoglobin genotypes HbAA, HbSS, HbSA, and HbSC, or HbA2. In other embodiments, the electrophoresis biochip can be used to diagnose whether the subject has SCD or an increased risk of SCD.

In some embodiments, the electrophoresis biochip can be used in a method where a blood sample from a subject is introduced into the sample loading port. The blood sample includes at least one blood cell. An electric field can be applied to the cellulose acetate paper and hemoglobin bands formed in the cellulose acetate paper are then imaged with the imaging system to determine hemoglobin phenotype for the subject. The hemoglobin phenotype can include HbAA, HbSA, HbSS, HbSC, or HbA2.

In some embodiment, an HbAA hemoglobin phenotype diagnoses the subject as normal, an HbSA hemoglobin phenotype diagnoses the subject as having a sickle cell trait, an HbSS hemoglobin phenotype diagnoses the subject as having a sickle cell disease, an HbSC hemoglobin phenotype diagnoses the subject as having a hemoglobin SC disease, and an HbA2 hemoglobin phenotype diagnoses the subject as having thalassemia.

Other embodiments described herein relate to a microfluidic biochip device that includes a housing. The housing includes at least one microchannel that defines at least one cell adhesion region. The at least one cell adhesion region is coated with at least one bioaffinity ligand that adheres a cell of interest when a fluid containing cells is passed through the at least one microchannel. The bioaffinity ligands can include at least one of fibronectin, laminin, selectin, von Willebrands' Factor, thrombomodulin or a C146 antibody. The device also includes an imaging system that measures the quantity of cells adhered to the at least one bioaffinity ligand within the at least one microchannel when the fluid is passed through the channels.

In some embodiments, the biochip device can quantitate membrane, cellular and adhesive properties of red blood cells and white blood cells of a subject to monitor disease severity, upcoming pain crisis, treatment response, and treatment effectiveness in a clinically meaningful way.

In some embodiments, the housing can include a plurality of microchannels. Each microchannel can include a separate cell adhesion region coated with at least one bioaffinity ligand. In other embodiments, at least two or at least three of the microchannels can include different bioaffinity ligands. In still other embodiments, the plurality of the microchannels can include the same bioaffinity ligands.

In some embodiments, at least one microchannel can include at least two different bioaffinity ligands coated on the cell adhesion region of the microchannel. The different bioffinity ligands can be located at different positions within the cell adhesion region of the at least one microchannel. For example, at least one of the laminin, the selectin, the von Willebrands' Factor, the thrombomodulin and the C146 antibody are localized at different positions along the at least one microchannel.

In some embodiments, the housing is a multilayer structure formed of a base layer, an intermediate layer, and a cover layer, the at least one microchannel can be formed in the intermediate layer. The housing can include an inlet port and an outlet port in fluid communication respectively with a first end and a second end of each microchannel. The at least one microchannel can be sized to accept microliter or milliliter volumes of blood or a solution containing cells to be adhered.

In some embodiments, the imaging system is a lensless imaging system. For example, the lensless imaging system can be a charged coupled device sensor and a light emitting diode.

In other embodiments, the cells are blood cells obtained from the subject and the imaging system can quantify the adhered cells in each respective channel to monitor the heath of a subject from which the cells are obtained. In other embodiments, the imaging system can quantify the adhered cells in each respective channel to monitor the progression of a disease, such as sickle cell disease, of a subject from which the cells are obtained. In still other embodiments, the imaging system can quantify the adhered cells in each channel to measure the efficacy of a therapeutic treatment administered to a subject from which the cells are obtained.

In some embodiments, the microfluidic biochip device can be used in a method where a blood sample comprising at least one blood cell from a subject is introduced into the microchannel and the quantity of cells adhered to the at least one bioaffinity ligand within the at least one microchannel is imaged. The biochip device can quantitate membrane, cellular and adhesive properties of red blood cells and white blood cells of a subject to monitor disease severity, upcoming pain crisis, treatment response, and treatment effectiveness in a clinically meaningful way.

## BRIEF DESCRIPTION OF THE FIGURES

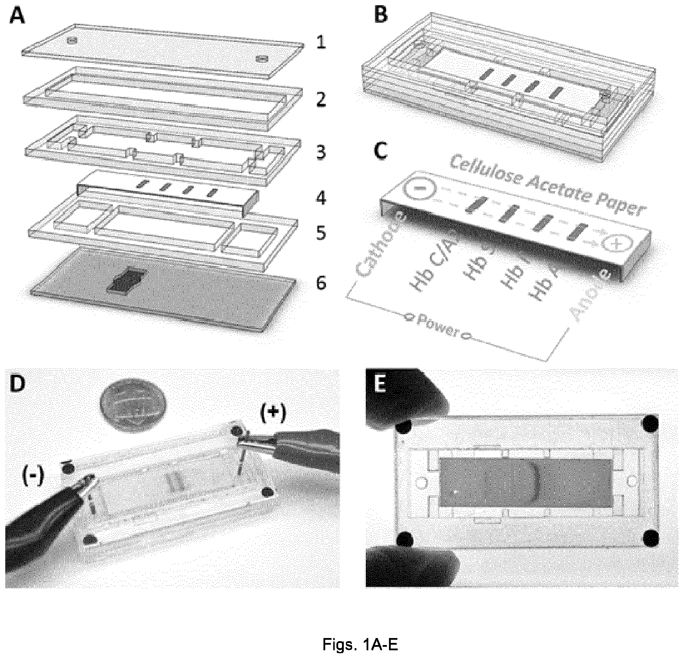

Figs. 1(A-E) illustrate a HemeChip for diagnosis of hemoglobin disorders, including sickle cell disease and thalassemias. (A) HemeChip is fabricated with multiple layer lamination of PMMA (1-3, 5-6) encompassing a single strip of cellulose acetate paper (4). (B) HemeChip has a compact design (5cm × 2cm × 0.6cm) and can be carried in a pocket. (C) Separation of all possible hemoglobin types is illustrated: Normal hemoglobin (Hb A0), Fetal hemoglobin (Hb F), Sickle hemoglobin (Hb S), and Hemoglobin C (Hb C) or A2. (D) Hemoglobin separation occurs in HemeChip via an applied electrical field, generated through graphite electrodes, between anode (+) and cathode (-). (E) A HemeChip prototype is shown with a miniscule amount of blood sample that has been separated into hemoglobin bands.

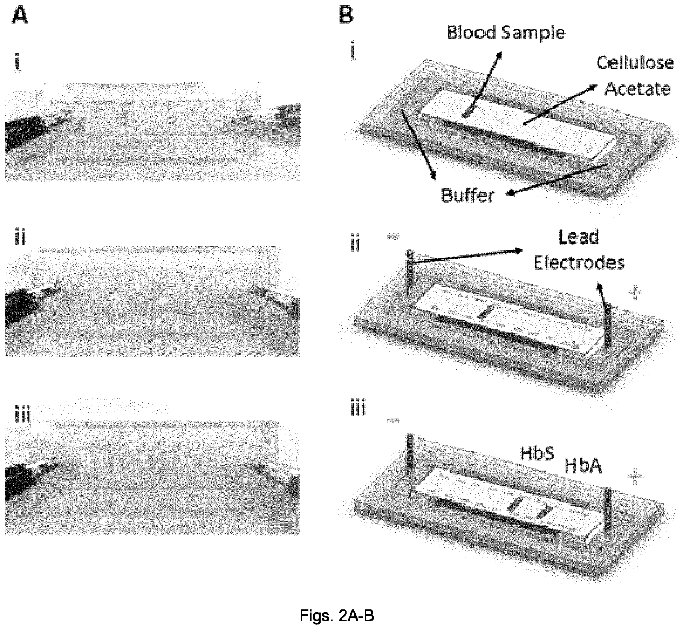

Figs. 2(A-B) illustrate images showing time lapse of microfluidic gel electrophoresis test. (A) Time lapse images from the HemeChip test performed, i) Beginning of test with blood added but no current yet applied, ii) Blood samples after 4 minutes with the current applied. iii) Blood samples after 8 minutes with the current applied. Current has been stopped and samples are in final positions. (B) Illustration of process of A with side view i) Beginning of test with blood added but no current yet applied. ii) Blood samples after 4 minutes with the current applied. iii) Blood samples after 8 minutes with the current applied. Current has been stopped and samples are in final positions.

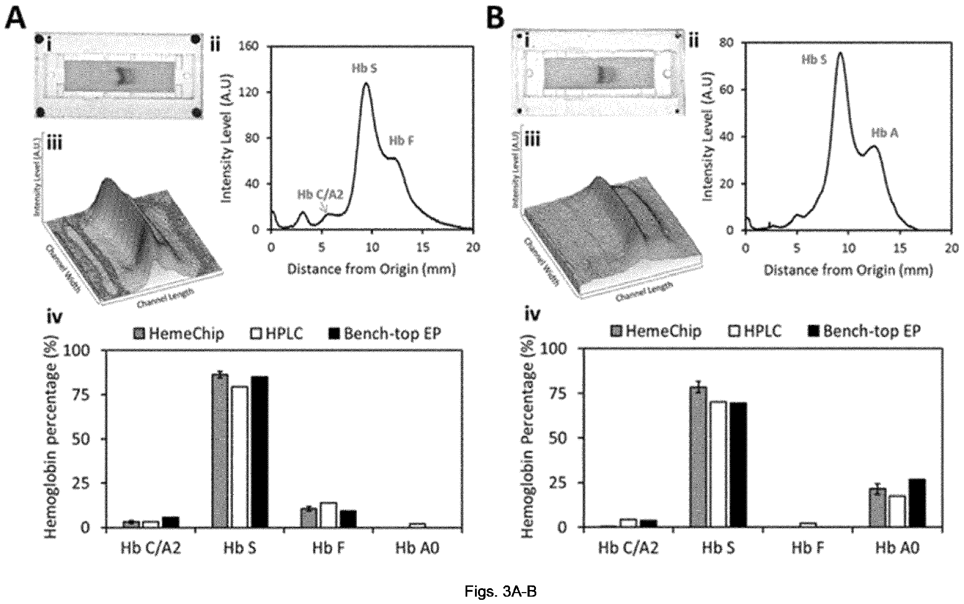

Figs. 3(A-B) illustrate identification and quantification of hemoglobin types in blood samples with different hemoglobin compositions using HemeChip. (A) An SS sample, SCD with hereditary persistence of fetal hemoglobin (HPFH), and (B) an SA sample, sickle cell trait (SCT) are shown. (i) Hemoglobin bands (stained with Ponceau S) formed in HemeChip allows identification of C/A2, S, F, and A0 hemoglobins. (ii) Intensities of each pixel measured and averaged through HemeChip length reveals intensity peaks identifying hemoglobin types A0 and S. Hb amount is quantified by calculating the area under the intensity peaks of each hemoglobin type (A.U. stands for arbitrary units), (iii) 3D surface profile of hemoglobin band pixel intensities obtained by image processing, (iv) Hemoglobin percentages measured using HemeChip, HPLC, and bench-top electrophoresis are compared.

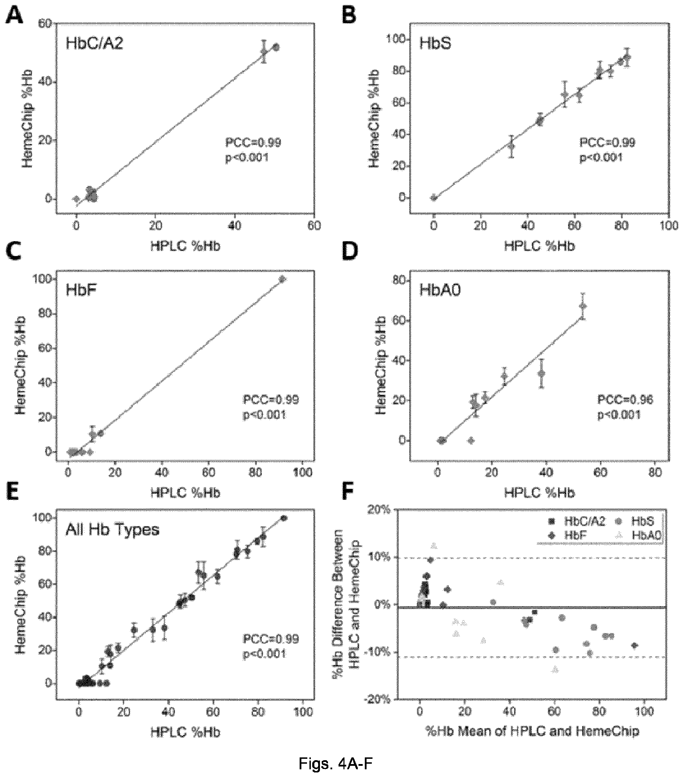

Figs. 4(A-F) illustrate the comparison of HemeChip hemoglobin quantification with the HPLC standard. (A-E) Correlation plots for comparison of HPLC to HemeChip hemoglobin quantification efficacy shows high positive correlations (PCC>0.96, p<0.001) for both individual and all hemoglobin types Hb C/A2, Hb S, Hb F, and Hb A0. (E) The data set consists of a total of 12 different patient blood samples (2x SS, 1× SS HPFH, 1× Cord Blood, 3x SC, 5x SA) and 43 individual experiments. (F) Bland-Altman plot shows strong agreement (SD difference=7%HbS) between estimated and actual %HbS (solid line = mean difference; dashed lines = 2 SD difference). The majority of the differences between actual and estimated %HbS (95.5%) are within 2 standard deviations of the mean of the differences. The hemoglobin types are indicated by dot color.

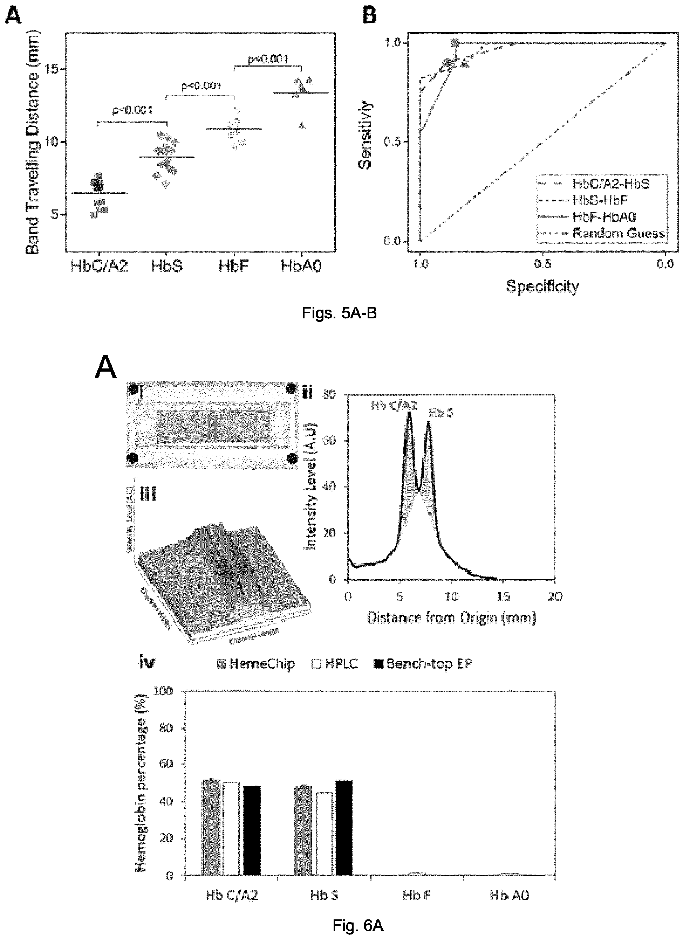

Figs. 5(A-B) illustrate distance travelled by each hemoglobin band. (A) The distance traveled from the application point in mm for each hemoglobin band under set conditions (250V, 1-2mA, for 8 min.). These distances will be used to mark the HemeChip and are used to identify the separation and presence of each hemoglobin type for overall diagnosis. The data set consists of 11 different patient blood samples (3x SS, 2x SS HPFH, 2x Cord Blood, 2x SC, 2x SA) and 32 experiments that produced multiple hemoglobin bands (20x Hb C/A2, 28x Hb S, 11× Hb F, and 7x Hb A0). The individual horizontal lines for each hemoglobin group represents the mean for the data set. The horizontal lines between hemoglobin groups represent statistically significant differences based on one way Analysis of Variance (ANOVA) test (p<0.001). (B) The receiver Operating-Characteristic (ROC) curve show sensitivity and specificity for differentiating between adjacent hemoglobin bands based on the travelling distance from the sample application point. Defined band traveling distance thresholds are shown (∘=7.5 mm, Δ=10.0 mm, and □=12.5 mm). For the defined thresholds, the ROC curve displayed more than 0.89 sensitivity and 0.86 specificity for identification of hemoglobin types.

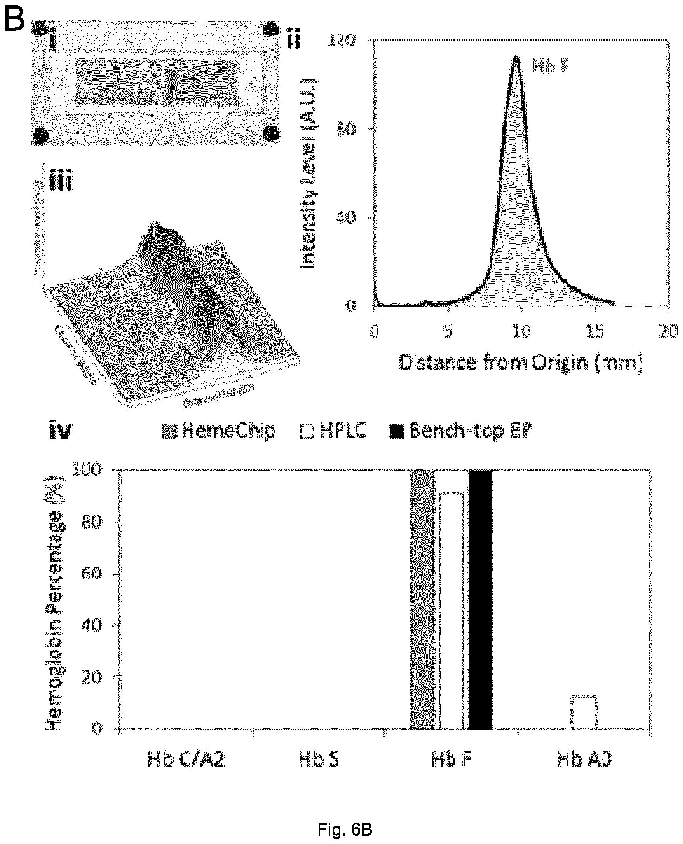

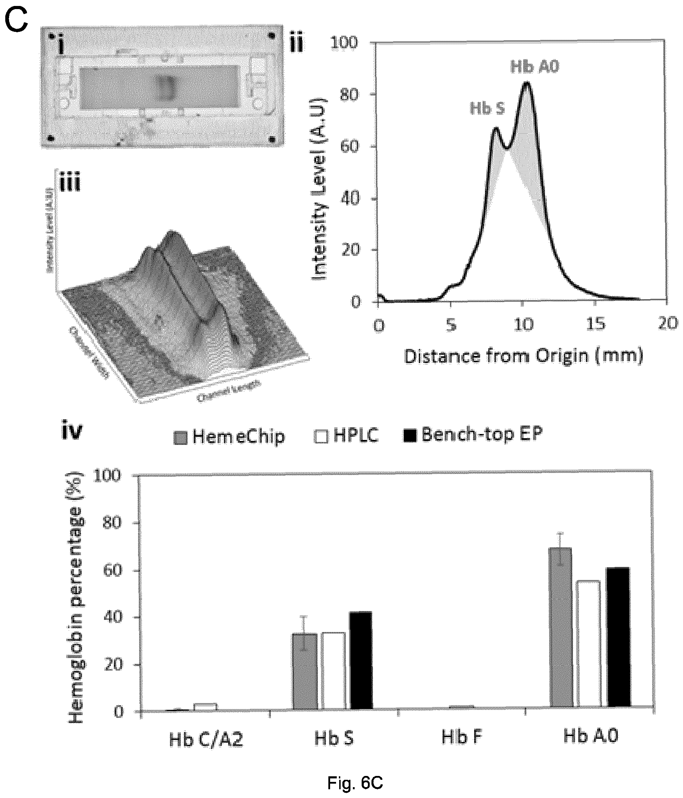

Figs. 6(A-C) illustrate additional sample analyses and comparisons with standard methods. Shaded red areas of the plot profiles (ii) represent the areas selected for hemoglobin quantification. (A) Sample with Hemoglobin SC disease (HbSC). (B) Cord blood with high HbF levels. (C) Sample with SCT (HbSA) with low levels of HbS and high levels of HbA0.

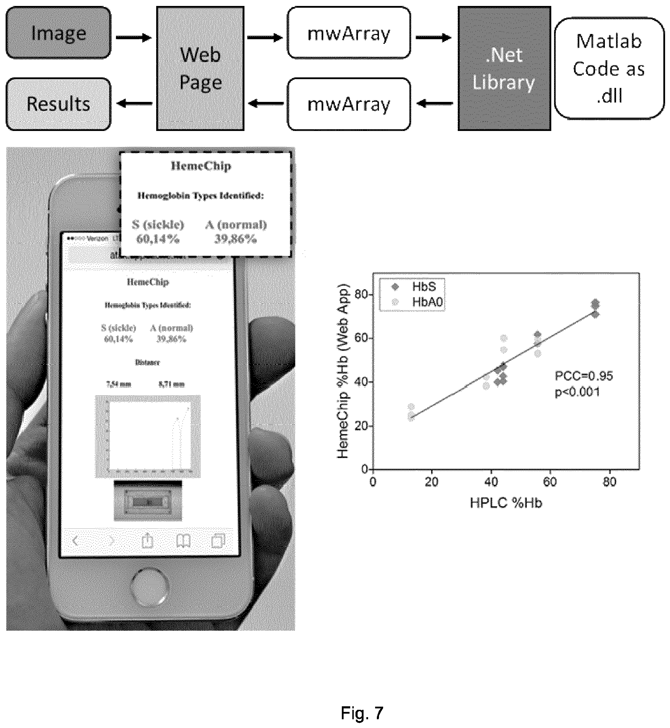

Fig. 7 illustrates a web-based image processing data flow and results comparison.

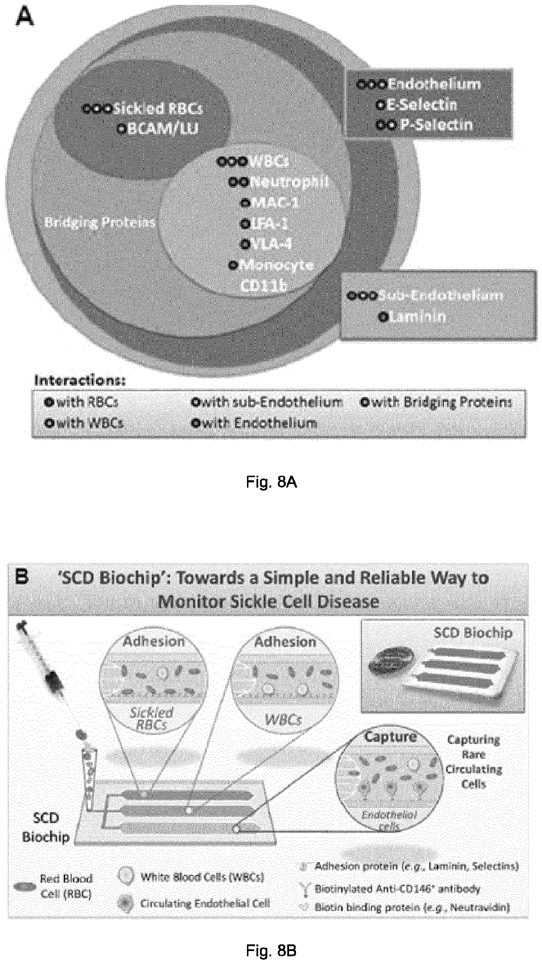

Figs. 8(A-B) illustrate a microfluidic biochip that evaluates cellular, membrane and adhesive (CMA) interactions. (A) Shown is a subset of potential interactions between cellular and sub-cellular components in SCD. Abnormal interactions may occur amongst: i) oxidized HbS containing RBCs; ii) soluble bridging proteins (i.e., for example thrombospondin (TSP) and/or von Willebrand Factor (vWF)); iii) cytokines and/or white blood cells (e.g., for example, CD11b+ monocytes); iv) activated endothelial cells comprising molecules including but not limited to, integrins, integrin receptors, adhesion molecules, and selectins; iv) subendothelial matrix components including but not limited to TSP, vWF, fibronectin, and laminin; and iv) activated WBCs (MAC-1+, LFA-1+, VLA-4+ neutrophils), which also directly adhere to the endothelium. (B) A schematic illustration of one embodiment for an SCD microfluidic biochip. Inset: photograph of a biochip comprising microfluidic channels filled with peripheral blood sample for analysis.

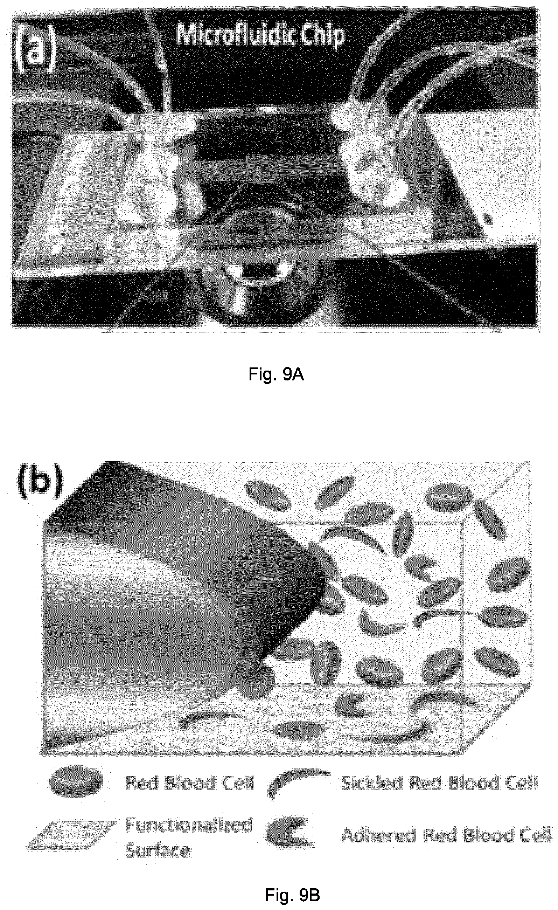

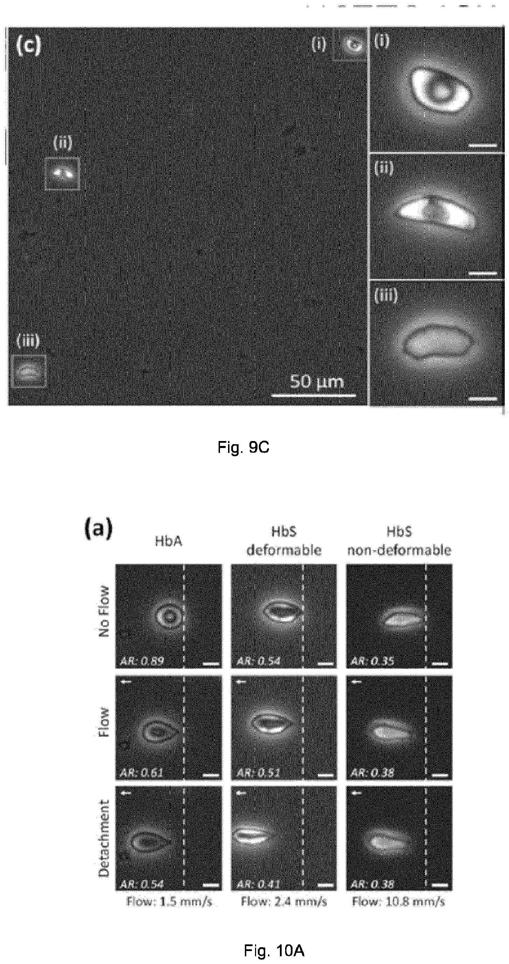

Figs. 9(A-C) illustrate an overview of a SCD microfluidic biochip system for evaluation of red blood cell (RBC) adhesion and deformability in physiological flow conditions. (A) A microfluidic biochip containing 50 $\mu$m high channels that can interrogate unprocessed whole blood. The microfluidic biochip is placed on an automated microscope stage for high resolution image recording and analysis of single RBCs. (B) Diagrammatic depiction of flowing and adhered RBCs on a fibronectin functionalized surface in the presence of 3D laminar flow velocity profile in the microfluidic biochip. (C) Exemplary data showing heterogeneity in adhered sickle RBC morphology as observed in microfluidic channels. RBCs from the same blood sample with different levels of sickling effect is shown: (i) mildly affected RBC, (ii) moderately affected RBC, and (iii) highly affected RBC. Scale bar represents 5 $\mu$m length.

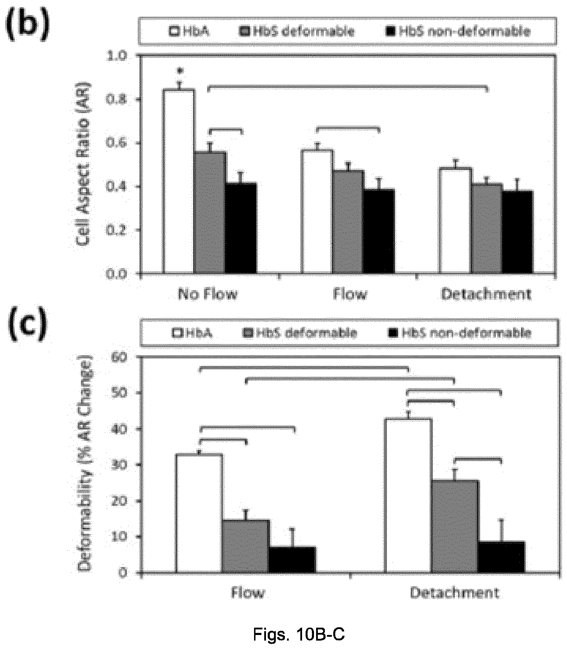

Figs. 10(A-C) illustrate exemplary data of aspect ratio (AR) and deformability of healthy (HbA-containing) and sickle RBCs (HbS-containing deformable and non-deformable) are presented under no flow, flow and detachment conditions. (A) Healthy and sickle RBCs at no flow, flow and detachment conditions are shown. Flow velocities that result in detachment of RBCs in different experimental groups are noted below each column. White dashed lines denote the initial positions of RBCs at no flow condition. Flow direction is denoted with arrowhead. Scale bar represents 5 $\mu$m length. Aspect Ratio (AR) of cells in each frame are provided at the lower left of the images. (B) Cell AR of different RBC groups are measured at no flow, flow, and detachment conditions. While HbA cells present a continuous decrease in AR from no flow to detachment, HbS non-deformable cells conserve their initial AR all the way through detachment. (C) Deformability (e.g., % cell AR change with respect to no flow condition) of healthy and sickle RBCs. Deformability of HbA and HbS deformable RBCs increases significantly from flow to detachment, whereas HbS non-deformable RBCs stay the same.

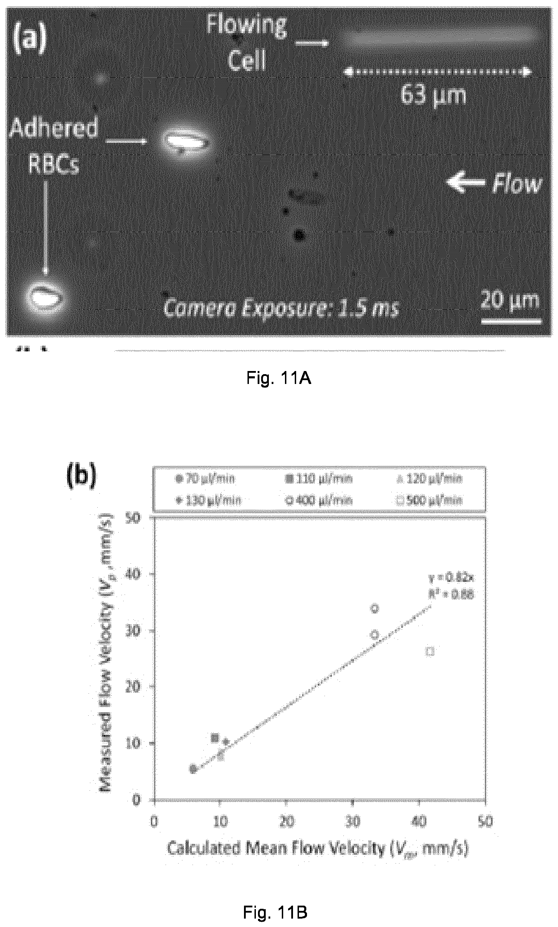

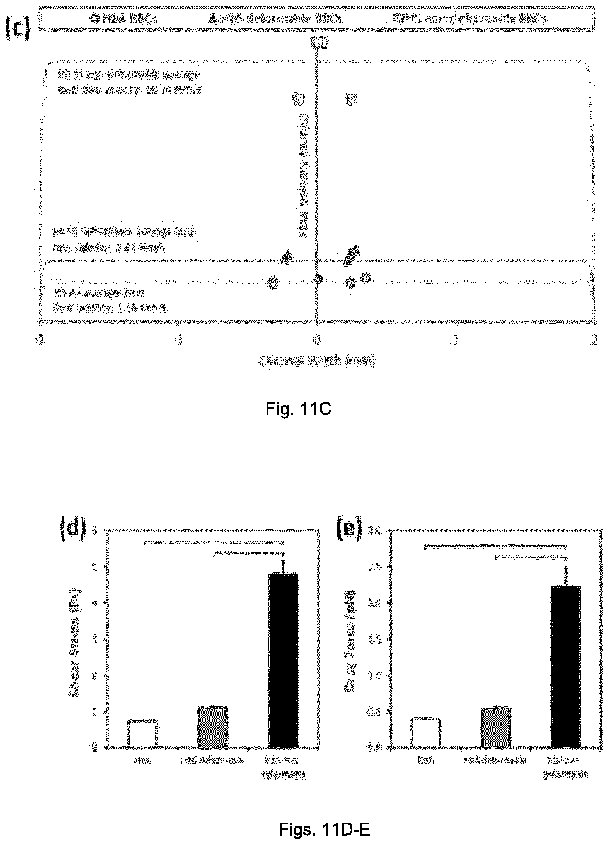

Figs. 11(A-E) illustrate exemplary data of HbS-containing non-deformable RBCs detachment at relatively higher flow velocity, shear stress, and drag force as compared with HbA and HbS-containing deformable RBCs. (A) Sequential images of RBCs during flow are recorded using an inverted microscope in phase contrast mode. Both adhered and free flowing RBCs are shown in the image analysis. (B) Shown is a correlation between the locally measured flow velocity and calculated mean flow velocity within the microchannels (Pearson correlation coefficient of 0.94, p<0.001). (C) Relative positions of adhered HbA, HbS non-deformable and HbS deformable RBCs in microchannels with respect to channel width (x- axis) and average local flow velocities (dotted lines) at detachment instant. HbS non-deformable RBCs are significantly different than HbA and HbS deformable RBCs in terms of flow velocity. (D) Relative differences in shear stress between HbA, HbS non-deformable and HbS deformable RBCs at detachment. (E) Relative differences in drag force between HbA, HbS non-deformable and HbS deformable RBC's at detachment.

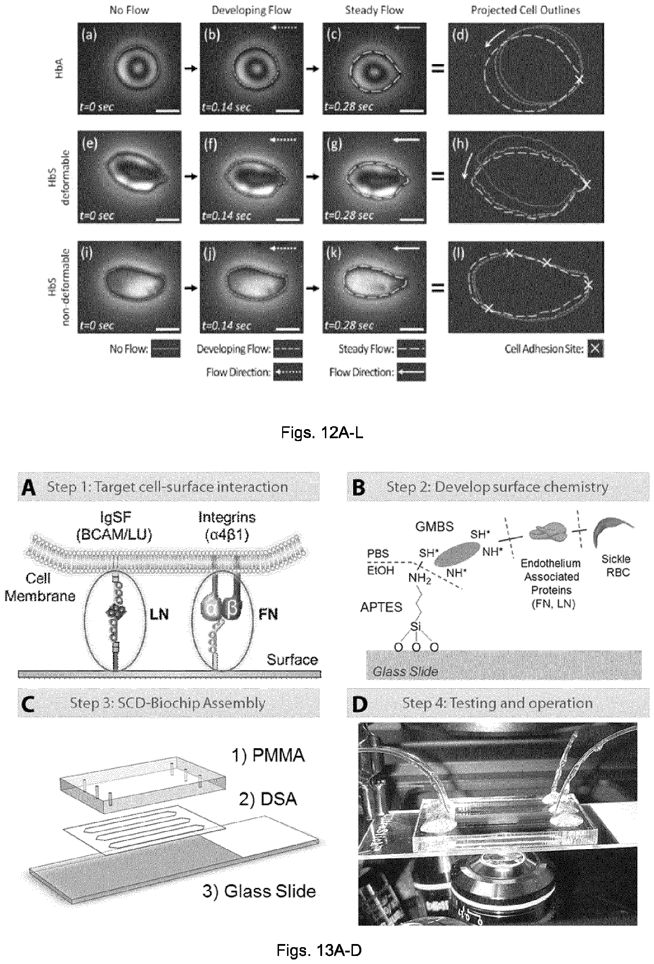

Figs. 12(A-L) illustrate exemplary data showing the determination of cell adhesion sites based on analysis of projected cell outlines at flow initiation for HbA- and HbS-containing RBCs. Outlines of individual RBCs in three consecutive frames taken over 0.28 seconds are projected to reflect the motion of the cells in response to initiation of fluid flow, for: (A-D) HbA-containing RBCs; (E-H) HbS-containing deformable RBCs; and (I-L) HbS-containing non-deformable RBCs.

Figs. 13(A-D) illustrate an overview of the development steps for the SCD-Biochip. (A) Adhesion receptors from the Immunoglobulin Superfamily (IgSF) BCAM/LU and integrin family ($\alpha4\beta1$) are targeted for adhesion to endothelial and sub-endothelial associated proteins, FN and LN. (B) FN and LN are covalently tethered to the glass slide through a cross linker (GMBS) and a self-assembled silane monolayer coating (APTES). (C) Assembly of the SCD Biochip, composed of a Polymethyl methacrylate (PMMA) cover, with micromachined inlets and outlets, a double sided adhesive (DSA) layer, which defines the channel shape and height, and a glass slide base. (D) SCD-Biochip placed on a microscope stage for live cell imaging.

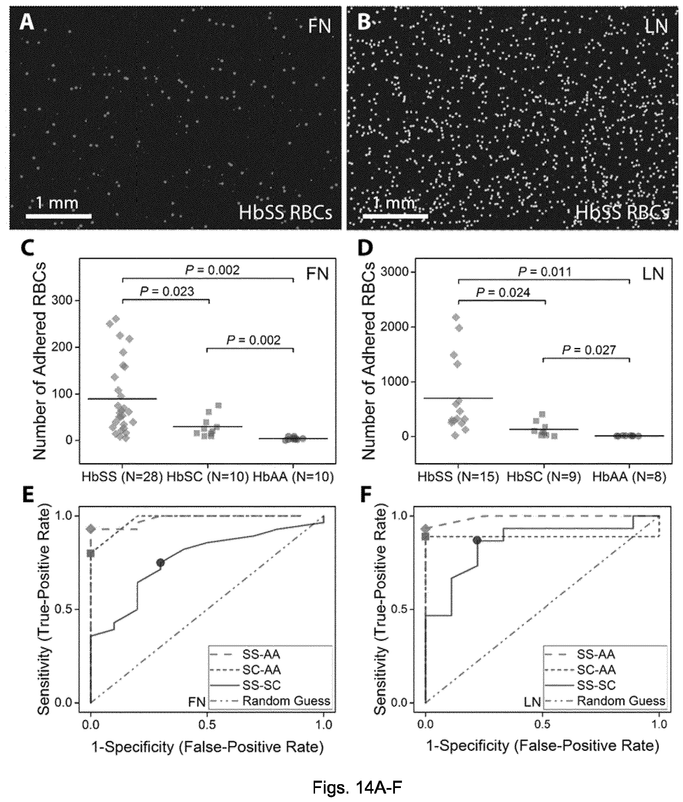

Figs. 14(A-F) illustrate exemplary data of variation of RBC adhesion in FN and LN functionalized microchannels amongst SCD hemoglobin phenotypes. (A-B) High resolution images of microchannels in FN or LN. (C-D) The number of adhered RBCs was significantly higher in samples from subjects with HbSS>HbSC/Sp+>HbAA in both FN and LN immobilized microchannels. The horizontal lines between individual groups represent a statistically significant difference based on a one-way ANOVA test (P<0.05). Data point cross bars represent the mean. 'N' represents the number of subjects. (E-F) Receiver Operating-Characteristic (ROC) curves display a true-positive rate (sensitivity) and a false-positive rate (1-specificity) for differentiation between SS-AA, SC-AA, and SS-SC hemoglobin phenotypes based on adhesion of RBCs on FN and LN. Defined thresholds for adhered RBC numbers on the ROC are as shown (◊=9, □=9, and o=30 for FN; ◊=16, □=16, and ∘=170 for LN). The ROC were strongest in discriminating between AA and SS or SC, compared with discrimination between SS and SC.

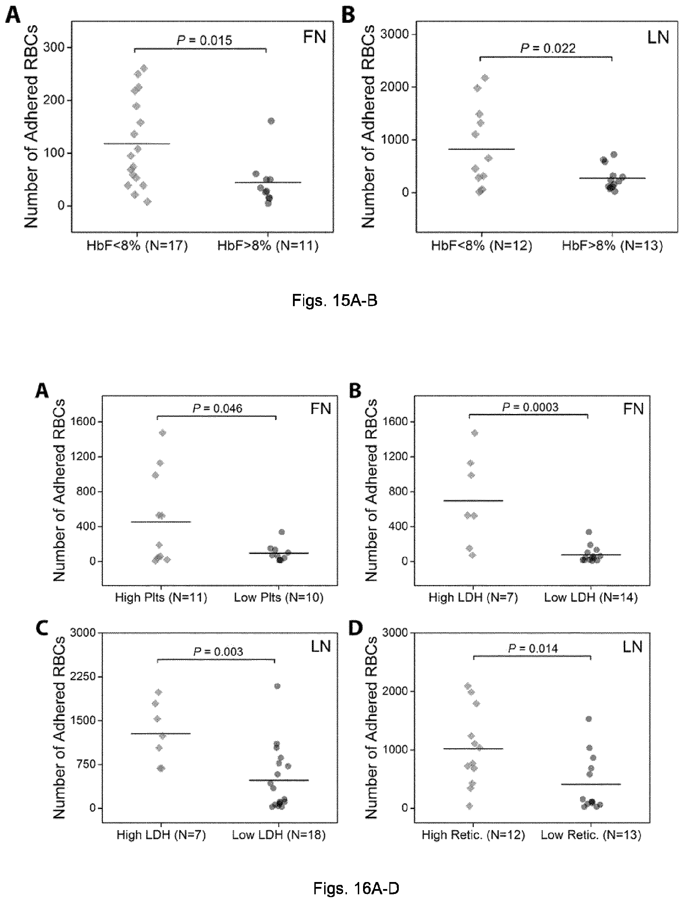

Figs. 15(A-B) illustrate exemplary data of RBC adhesion of RBCs is greater in HbSS subjects with a low (<%8) HbF, compared with high (>%8) HbF. (A-B) RBC adhesion was quantified in blood samples of HbSS patients with high and low HbF levels. Number of adhered RBCs was significantly higher in blood samples from subjects with low HbF levels compared to blood samples from subjects with high HbF levels in both FN and LN immobilized microchannels. The horizontal lines between individual groups represent a statistically significant difference based on a one way ANOVA test (P<0.05). Data point cross bars represent the mean. 'n' represents the number of subjects.

Figs. 16(A-D) illustrate exemplary data of association between RBC adhesion and lactate dehydrogenase (LDH), platelet counts (plts), and reticulocyte counts (retics) in HbSS. (A-B) Number of adhered RBCs in FN microchannels was significantly higher in blood samples with (A) high LDH (>500 U/L) and (B) high plts (>320 109/L), respectively. (C-D) RBCs in blood samples with (C) high LDH (>500 u/L) and (D) retics (>320 109/L) showed a significantly higher adherence to LN immobilized microchannels compared to low LDH (<500 U/L) and low retics (<320 109/L), respectively. The horizontal lines between individual groups represent a statistically significant difference based on a one way ANOVA test (P<0.05). Data point cross bars represent the mean. 'n' represents the number of subjects.

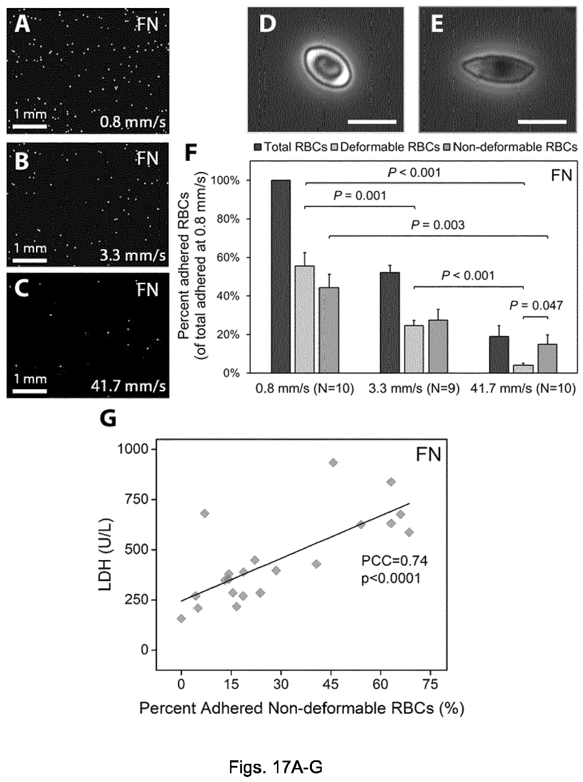

Figs. 17(A-G) illustrate exemplary data of heterogeneity in adhered RBCs in FN functionalized microchannels and its association with serum LDH levels. (A-C) Number of adhered RBCs and morphologies were analyzed in HbSS blood samples at step-wise increased flow velocities; (A) 0.8 mm/s, (B) 3.3 mm/s, and (C) 41.7 mm/s. (D-E) Deformable (with characteristic biconcave shape) and non-deformable RBCs (without characteristic biconcave shape) were determined based on morphological characterization. Scale bars represent a length of 5 $\mu$m. (F) Percentages of deformable and non-deformable RBCs of total adhered RBCs at 0.8 mm/s flow velocity were calculated. There was no significant difference between percentages of deformable and non-deformable RBCs at 0.8 mm/s flow velocity (P=0.266), however, the percentage of non-deformable RBCs was significantly higher than that of deformable RBCs at 41.7 mm/s flow velocity. The horizontal lines between individual groups represent a statistically significant difference based on a one way ANOVA test (P<0.05). Error bars represent the standard error of the mean. 'n' represents the number of subjects. (G) A statistically significant correlation was observed between the percentage of adhered non-deformable RBCs and LDH at 0.8 mm/s (Pearson correlation coefficient of 0.79, n=21 samples).

## DETAILED DESCRIPTION

Definitions

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity but also plural entities and also includes the general class of which a specific example may be used for illustration. The terminology herein is used to describe specific embodiments of the invention, but their usage does not delimit the invention, except as outlined in the claims.

The term "microchannels" as used herein refer to pathways through a medium (e.g., silicon) that allow for movement of liquids and gasses. Microchannels thus can connect other components, i.e., keep components "in liquid communication." While it is not intended that the present invention be limited by precise dimensions of the channels, illustrative ranges for channels are as follows: the channels can be between 0.35 and 100 $\mu$m in depth (preferably 50 $\mu$m) and between 50 and 1000 $\mu$m in width (preferably 400 $\mu$m). Channel length can be between 4 mm and 100 mm, or about 27 mm. An "electrophoresis channel" is a channel substantially filled with a material (e.g., an agarose gel, polyacrylamide gel, cellulose acetate paper) that aids in the differential migration of biological substances (e.g., for example whole cells, proteins, lipids, nucleic acids). In particular, an electrophoresis channel may aid in the differential migration of blood cells based upon mutations in their respective hemoglobin content.

The term "microfabricated", "micromachined" and/or "micromanufactured" as used herein, means to build, construct, assemble or create a device on a small scale (e.g., where components have micron size dimensions) or microscale. In one embodiment, electrophoresis devices are microfabricated ("microfabricated electrophoresis device") in about the millimeter to centimeter size range.

The terms "agarose gel", "polyacrylamide gel", and "cellulose acetate" are terms understood by those practiced in the art to mean a medium that suppresses convective mixing of the fluid phase through which electrophoresis takes place and contributes molecular sieving. For example, a polyacrylamide gels may be crosslinked or non-crosslinked. The term "crosslinked" means the linking of the chains of a polymer (e.g., polyacrylamide) to one another so that the polymer, as a network, becomes stronger and more resistant to being dissolved and permits better separation of sample components when used in electrophoresis. Bis-acrylamide is an example of a cross-linking agent used in polyacrylamide electrophoresis.

The term "polymer" refers to a substance formed from two or more molecules of the same substance. Examples of a polymer are gels, crosslinked gels and polyacrylamide gels. Polymers may also be linear polymers. In a linear polymer the molecules align predominately in chains parallel or nearly parallel to each other. In a non-linear polymer the parallel alignment of molecules is not required.

The term "microelectrophoresis device" as used herein, refers to a small (*e.g.,* micron size components) scale device for performing electrophoresis. In one embodiment, it is contemplated that the microelectrophoresis device comprises electrophoresis channels of about 4000 $\mu$m or less (width) by 2000 $\mu$m or less (depth).

The term "electrode" as used herein, refers to an electric conductor through which an electric current enters or leaves, for example, an electrophoresis gel or other medium.

The term "channel spacer" as used herein, refers to a solid substrate capable of supporting lithographic etching. A channel spacer may comprise one, or more, microchannels and is sealed from the outside environment using dual adhesive films between a top cap and a bottom cap, respectively.

The term "lensless imaging system" as used herein, refers to an optical configuration that collects an image based upon electronic signals as opposed to light waves. For example, a lensless image may be formed by excitation of a charged coupled device sensor (CCD) by emissions from a light emitting diode.

The term "charge-coupled device (CCD)" as used herein, refers to a device for the movement of electrical charge, usually from within the device to an area where the charge can be manipulated, for example, a conversion into a digital value. CCD provide digital imaging when using a CCD image sensor where pixels are represented by p-doped MOS capacitors.

The term "suspected of having", as used herein, refers a medical condition or set of medical conditions (*e.g.*, preliminary symptoms) exhibited by a patient that is insufficient to provide a differential diagnosis. Nonetheless, the exhibited condition(s) would justify further testing (*e.g.*, autoantibody testing) to obtain further information on which to base a diagnosis.

The term "at risk for" as used herein, refers to a medical condition or set of medical conditions exhibited by a patient which may predispose the patient to a particular disease or affliction. For example, these conditions may result from influences that include, but are not limited to, behavioral, emotional, chemical, biochemical, or environmental influences.

The term "symptom", as used herein, refers to any subjective or objective evidence of disease or physical disturbance observed by the patient. For example, subjective evidence is usually based upon patient self-reporting and may include, but is not limited to, pain, headache, visual disturbances, nausea and/or vomiting. Alternatively, objective evidence is usually a result of medical testing including, but not limited to, body temperature, complete blood count, lipid panels, thyroid panels, blood pressure, heart rate, electrocardiogram, tissue and/or body imaging scans.

The term "disease" or "medical condition", as used herein, refers to any impairment of the normal state of the living animal or plant body or one of its parts that interrupts or modifies the performance of the vital functions. Typically manifested by distinguishing signs and symptoms, it is usually a response to: i) environmental factors (as malnutrition, industrial hazards, or climate); ii) specific infective agents (as worms, bacteria, or viruses); iii) inherent defects of the organism (as genetic anomalies); and/or iv) combinations of these factors.

The term "patient" or "subject", as used herein, is a human or animal and need not be hospitalized. For example, out-patients, persons in nursing homes are "patients." A patient may comprise any age of a human or non-human animal and therefore includes both adult and juveniles (*i.e.,* children). It is not intended that the term "patient" connote a need for medical treatment, therefore, a patient may voluntarily or involuntarily be part of experimentation whether clinical or in support of basic science studies.

The term "affinity" as used herein, refers to any attractive force between substances or particles that causes them to enter into and remain in chemical combination. For example, an inhibitor compound that has a high affinity for a receptor will provide greater efficacy in preventing the receptor from interacting with its natural ligands, than an inhibitor with a low affinity.

The term "derived from" as used herein, refers to the source of a compound or sample. In one respect, a compound or sample may be derived from an organism or particular species.

The term "antibody" refers to immunoglobulin evoked in animals by an immunogen (antigen). It is desired that the antibody demonstrates specificity to epitopes contained in the immunogen. The term "polyclonal antibody" refers to immunoglobulin produced from more than a single clone of plasma cells; in contrast "monoclonal antibody" refers to immunoglobulin produced from a single clone of plasma cells.

The terms "specific binding" or "specifically binding" when used in reference to the interaction of an antibody and a protein or peptide means that the interaction is dependent upon the presence of a particular structure (*i.e.,* for example, an antigenic determinant or epitope) on a protein; in other words an antibody is recognizing and binding to a specific protein structure rather than to proteins in general. For example, if an antibody is specific for epitope "A", the presence of a protein containing epitope A (or free, unlabeled A) in a reaction containing labeled "A" and the antibody will reduce the amount of labeled A bound to the antibody.

The term "sample" as used herein is used in its broadest sense and includes environmental and biological samples. Environmental samples include material from the environment such as soil and water. Biological samples may be animal, including, human, fluid (e.g., blood, plasma and serum), solid (e.g., stool), tissue, liquid foods (e.g., milk), and solid foods (e.g., vegetables). A biological sample may comprise a cell, tissue extract, body fluid, chromosomes or extrachromosomal elements isolated from a cell, genomic DNA (in solution or bound to a solid support such as for Southern blot analysis), RNA (in solution or bound to a solid support such as for Northern blot analysis), cDNA (in solution or bound to a solid support) and the like.

The terms "bioaffinity ligand", "binding component", "molecule of interest", "agent of interest", "ligand" or "receptor" as used herein may be any of a large number of different molecules, biological cells or aggregates, and the terms are used interchangeably. Each binding component may be immobilized on a solid substrate and binds to an analyte being detected. Proteins, polypeptides, peptides, nucleic acids (nucleotides, oligonucleotides and polynucleotides), antibodies, ligands, saccharides, polysaccharides, microorganisms such as bacteria, fungi and viruses, receptors, antibiotics, test compounds (particularly those produced by combinatorial chemistry), plant and animal cells, organelles or fractions of each and other biological entities may each be a binding component. Each, in turn, also may be considered as analytes if same bind to a binding component on a microfluidic biochip.

The terms "bind" or "adhere" as used herein, include any physical attachment or close association, which may be permanent or temporary. Generally, an interaction of hydrogen bonding, hydrophobic forces, van der Waals forces, covalent and ionic bonding etc., facilitates physical attachment between the molecule of interest and the analyte being measuring. The "binding" interaction may be brief as in the situation where binding causes a chemical reaction to occur. That is typical when the binding component is an enzyme and the analyte is a substrate for the enzyme. Reactions resulting from contact between the binding agent and the analyte are also within the definition of binding for the purposes of the present invention.

The term, "substrate" as used herein, refers to surfaces as well as solid phases which may comprise a microchannel. In some cases, the substrate is solid and may comprise PDMS. A substrate may also comprise components including, but not limited to, glass, silicon, quartz, plastic, or any other composition capable of supporting photolithography.

The term, "photolithography", "optical lithography" or "UV lithography" as used herein, refers to a process used in microfabrication to pattern parts of a thin film or the bulk of a substrate. It uses light to transfer a geometric pattern from a photomask to a light-sensitive chemical "photoresist", or simply "resist," on the substrate. A series of chemical treatments then either engraves the exposure pattern into, or enables deposition of a new material in the desired pattern upon, the material underneath the photo resist. For example, in complex integrated circuits, a modern CMOS wafer will go through the photolithographic cycle up to 50 times.

Embodiments described herein relate to biochips and to the use of biochips to rapidly and easily diagnose hemoglobin disorders, such as hemoglobinopathies (e.g., sickle cell disease (SCD)), and to quantitate membrane, cellular and adhesive properties of blood cells, such as red blood cells and white blood cells, of a subject to monitor disease severity, upcoming pain crisis, treatment response, and treatment effectiveness in a clinically meaningful way.

Sickle Cell Disease (SCD)

SCD is believed to be an inherited blood disorder, which may result from a single mutation in the hemoglobin gene. Anemia resulting from 'sickle-shaped" hemoglobin was first clinically described in the United States in 1910, and a mutated heritable sickle hemoglobin molecule was identified in 1949. Herrick J., "Peculiar elongated and sickle-shaped red blood cell corpuscles in a case of severe anemia" Archives of Internal Medicine 1910; and Pauling et al., "Sickle cell anemia, a molecular disease" Science 109:443 1949.

Hemoglobin is a protein in red blood cells that carries oxygen. It is believed that the pathophysiology of SCD is a consequence of abnormal polymerization of deoxygenated sickled hemoglobin that affects red cell membrane properties, shape, density, and/or integrity. It is further believed that these effects lead to changes in inflammatory cell and endothelial cell function. Some clinical consequences of SCD may include, but are not limited to, painful crises, widespread organ damage, and early mortality.

Normal hemoglobin (HbA) is usually comprised of alpha and beta chains. Individuals who inherit one copy of a sickle mutated beta chain and one copy of a normal beta chain have a sickle cell trait (HbAS). These people are healthy but may unknowingly transmit SCD to their children. Individuals who inherit two copies of a sickle mutated beta chain (HbSS) develop SCD. Individuals who carry one copy of the sickle chain and one copy of the abnormal beta chain C have compound heterozygous HbSC. HbSC confers a milder, but still debilitating form of SCD. Nagel et al., "The paradox of hemoglobin SC disease" Blood Reviews 17:167-178 (2003). Of the current diagnosed SCD population approximately two-thirds are believed to be homozygous HbSS and one-quarter have the compound heterozygous HbSC trait. The remaining SCD population is comprised of patients with HbSbeta thalassemia, in which HbS and small (or no) amounts of HbA are made.

SCD is a recognized molecular disease and is caused by a mutation of the beta globin gene in hemoglobin. Pauling et al., Science 110(2865):543-548 (1949). Replacement of a hydrophilic amino acid with a hydrophobic amino acid in the 6th position of the β-globin chain leads to polymerization of intracellular HbS and to the formation of stiff hemoglobin polymer structures within the cell. Barabino et al., Annual review of biomedical engineering 12:345-367 (2010). This mutation afflicts millions of people worldwide and is associated with considerable morbidity and mortality. Platt et al., N. Engl. J. Med. 330(23):1639-1644 (1994).

The pathophysiology of SCD is a consequence of abnormal hemoglobin polymerization and its deleterious effects on RBC membrane, shape, density, adhesion, and deformability. Hillery et al., Blood 87(11):4879- 4886 (1996); Kaul et al., Microcirculation 16(1):97-111 (2009); Kaul et al., Proceedings of the National Academy of Sciences of the United States of America 86(9):3356-3360 (1989); Hebbel et al., The New England journal of medicine 302(18):992-995 (1980); Stuart et al., 364(9442):1343-1360 (2004); and Hoover et al., Blood 54(4):872-876 (1979). A healthy RBC has a characteristic biconcave shape that allows cells to easily deform and pass through minuscule vessels and capillaries in the body. However, sickled RBCs undergo a radical morphological transformation that leads to reduced deformability, increased stiffness, and abnormal adhesion causing a blockage of blood vessels known as vaso-occlusion. The consequences of this blood vessel blockade include painful crises, wide-spread organ damage, and early mortality. An et al., British journal of haematology 141(3):367-375 (2008); Mohandas et a1., Blood 112(10):3939-3948 (2008); Lipowsky, H. H., Microcirculation 12(1):5-15 (2005); and Hofrichter et a1., Proceedings of the National Academy of Sciences of the United States of America 71(12):4864-4868 (1974).

Red Blood Cell (RBC) deformability, adhesion, hemolysis, and alterations in flow are believed to be pathophysiologic symptoms of Sickle Cell Disease (SCD). Barabino et a1., Annual Review Of Biomedical Engineering 12:345-367 (2010); and Alexy et a1., Transfusion 46(6):912-918 (2006). Many facets of sickle RBCs have been investigated, including hemoglobin polymerization, cellular deformability, adhesion, hemodynamic changes, and/or clinical heterogeneity. Hebbel R. P., Blood 77(2):214-237 (1991); Ferrone, Microcirculation 11(2):115-28 (2004); Noguchi et a1., Annual review of biophysics and biophysical chemistry 14:239-263 (1985); Nash et a1., Blood 63(1):73-82 (1984); Brandao et a1., European journal of haematology 70(4):207-211 (2003); Mohandas et a1., Journal of Clinical Investigation 76(4):1605-1612 (1985); Montes et a1., American journal of hematology 70(3):216-227 (2002); Hillery et al., Blood 87(11):4879-4886 (1996); Kasschau et a1., Blood 87(2):771-780 (1996); Bartolucci et a1., Blood 120(15):3136-3141 (2012); Kaul et a1., Microcirculation 16(1):97-111 (2012); Kaul et a1., The Journal of clinical investigation 72(1):22-31 (1983); Lei et a1., Proceedings of the National Academy of Sciences of the United States of America 110(28):11326-11330 (2013); Bunn et a1., The New England journal of medicine 337(11):762-769 (1997); Ballas et a1., Microcirculation 11(2):209-225 (2004); and Kaul et a1., Proceedings of the National Academy of Sciences of the United States of America 86(9):3356-3360 (1989).

Specifically, sickled RBC adhesion (i.e., for example, stickiness) and deformability have been identified as biophysical factors involved in vaso-occlusion and have been shown to correlate with SCD severity. Hebbel et a1., Journal of Clinical Investigation 100(11):S83-S86 (1997); Hebbel et a1., The New England journal of medicine 302(18):992-995 (1980); and Ballas et a1., Blood 79(8):2154-2163 (1992). These factors have not been studied in isolation due to significant technological barriers faced in directly analyzing blood samples from diverse clinical phenotypes. This challenge has posed significant limitations to the cogent integration of complex biophysical phenomena into an understanding of a heterogeneous multi-faceted clinical condition, such as SCD.

Dramatic changes in morphologic, physical, and hemodynamic properties of RBCs are caused by HbS polymerization. Barabino et al., Annual review of biomedical engineering 12:345-367 (2010). Earlier studies have shown that RBCs in SCD patients are heterogeneous in density, morphology, and function. Kaul et al., The Journal of clinical investigation 72(1):22-31 (1983), Fabry et al., Blood 60(6):1370-1377 (1982); Kaul et al., Microvascular research 26(2): 170-181 (1983); Lipowsky et al., The Journal of clinical investigation 80(1):117-127 (1987); and Kaul et al., Blood 68(5):1162-1166 (1986). The data presented herein show that HbS-containing RBCs also vary in deformability, adhesion strength, and in the number of adhesion sites. In addition, deformable HbS-containing RBCs are less adherent, while non-deformable cells are more adherent to fibronectin (FN). In a recent study, decreased deformability and RBC aggregation, measured using ektacytometry and laser back scatter of Percoll-separated sickle RBCs, were shown to correlate with hemolysis. Connes et al., British journal of haematology 165(4):564-572 (2014). Consequently, it is believed that RBC adhesion and deformability are components of vasoocclusion and hemolysis in subjects with SCD.

Non-beta chain hemoglobinopathies, such as alpha thalassemia, are also major world-wide health problems in children, especially in Asia. It is expected that, in some embodiments, the present invention can be used to test this population, once the electrophoretic diagnosis of beta-chain abnormalities, such as HbSS and HbSC have been established. It is also likely that this technology will be adaptable to the need for detecting additional mutant hemoglobins, such as Hemoglobin Bart's (4 fetal beta-type gamma chains) or HbH (4 adult beta-type chains), which are elevated in alpha thalassemia.

Pathophysiologic changes in SCD include, but are not limited to, alterations in adhesion amongst sickled red blood cells (RBCs), activated white blood cells (WBCs), activated endothelium, abnormal numbers of circulating endothelial cells and abnormal numbers of circulating hematopoietic precursor cells. (Fig. 8A). However, observed pathophysiologic correlates have not been widely tested, due to technical limitations and only localized expertise.

It has been reported that abnormal adherence to endothelium by sickled RBCs and WBCs in SCD may be a possible cause of pain and vasculopathy mediated by inflammation and abnormal cellular adhesion. Hebbel et al., "Erythrocyte adherence to endothelium in sickle-cell anemia. A possible determinant of disease severity" N Engl J Med 302:992-995 (1980); Hebbel et al., "Erythrocyte/endothelial interactions and the vasocclusive severity of sickle cell disease" Prog Clin Biol Res 55:145-162 (1981); Barabino et al., "Endothelial cell interactions with sickle cell, sickle trait, mechanically injured, and normal erythrocytes under controlled flow" Blood 70:152-157 (1987); and Barabino et al., "Rheological studies of erythrocyteendothelial cell interactions in sickle cell disease" Prog Clin Biol Res 240:113-127 (1987). A myriad of interconnecting abnormal interactions can be envisioned, amongst HbS-containing RBCs, activated WBCs, and activated endothelial cells, resulting in pain and devastation in SCD. (Fig. 8A). Abnormal monocyte, neutrophil, platelet, and endothelial cell activation and adhesion may be present in SCD, and complementary models of vasoocclusive crises describe initial reticulocyte and neutrophil adhesion to an activated endothelium and/or subendothelial matrix *(i.e.,* for example, comprising LN, FN, vWF), followed by dense (irreversibly sickled) red cell trapping and vaso-occlusion. Setty et al., "Role of erythrocyte phosphatidylserine in sickle red cell endothelial Adhesion" Blood 99:1564-1571 (2002); Kaul et al., "Sickle red cell-endothelium interactions" Microcirculation 16:97-111 (2009); and Frenette et al., "Sickle cell disease: old discoveries, new concepts, and future promise" J. Clin Invest 117:850-858 (2007).

Alternatively, *ex vivo* and *in vivo* experiments have suggested that the endothelium is activated by cytokines and white cells, primarily monocytes, which are themselves activated by sickle RBC-derived factors. Belcher et al., "Activated monocytes in sickle cell disease: potential role in the activation of vascular endothelium and vaso-occlusion" Blood 96:2451-2459 (2000); Natarajan et al., "Adhesion of sickle red blood cells and damage to interleukin-1 beta stimulated endothelial cells under flow in vitro" Blood 87:4845-4852 (1996); Perelman et al., "Placenta growth factor activates monocytes and correlates with sickle cell disease severity" Blood 102:1506-1514 (2003); and Zennadi et al., "Sickle red cells induce adhesion of lymphocytes and monocytes to endothelium" Blood 112:3474-3483 (2008). These factors combine to increase the adhesiveness of RBCs and white cells, primarily neutrophils, to each other and to the endothelium and subendothelium. Soluble bridging factors *(i.e.,* for example, TSP, FN, and vWF) may also be involved, although these interactions have not been quantified. Stone et al., "Effects of density and of dehydration of sickle cells on their adhesion to cultured endothelial cells" Am J Hematol 52:135-143 (1996); Kaul et a1., "Sickle erythrocyte-endothelial interactions in microcirculation: the role of von Willebrand factor and implications for vasoocclusion" Blood 81:2429-2438 (1993): Wick et a1., "Unusually large von Willebrand factor multimers increase adhesion of sickle erythrocytes to human endothelial cells under controlled flow" J Clin Invest 80:905-910 (1987); Brittain et a1., "Thrombospondin from activated platelets promotes sickle erythrocyte adherence to human microvascular endothelium under physiologic flow: a potential role for platelet activation in sickle cell vaso-occlusion" Blood 81:2137-2143 (1993); Sugihara et a1., "Thrombospondin mediates adherence of CD36+ sickle reticulocytes to endothelial cells" Blood 80:2634-2642 (1992); Barabino et a1., "Inhibition of sickle erythrocyte adhesion to immobilized thrombospondin by von Willebrand factor under dynamic flow conditions" Blood 89:2560-2567 (1997); Finnegan et a1., "Adherent leukocytes capture sickle erythrocytes in an in vitro flow model of vaso-occlusion" Am J Hematol 82:266-275 (2007); Turhan et a1., "Primary role for adherent leukocytes in sickle cell vascular occlusion: a new paradigm" Proc Natl Acad Sci U S A 99:3047-3051 (2002). Further, activated endothelial cells and hematopoietic precursor cells circulate at an unusually high level in SCD and correlate with end-organ damage. Some membrane/cellular interactions have been studied during vasoocclusive crises or compellingly demonstrated in animal models, but broad clinically correlative studies are absent. Hebbel et a1., "Erythrocyte adherence to endothelium in sickle-cell anemia" New Engl J Med 302:992-995 (1980); Solovey et a1., "Circulating activated endothelial cells in sickle cell anemia" N Engl J Med 337:1584-1590 (1997); Strijbos et a1., "Circulating endothelial cells: a potential parameter of organ damage in sickle cell anemia?" Blood Cells Mol Dis 43:63-67 (2009); van Beem et a1., "Elevated endothelial progenitor cells during painful sickle cell crisis" Exp Hematol 37:1054-1059 (2009); and Jang et a1., "CXCL1 and its receptor, CXCR2, mediate murine sickle cell vaso-occlusion during hemolytic transfusion reactions" J Clin Invest 121:1397-1401.

Red Blood Cell Adhesion

In all vertebrates, with the exception of white-blooded fish (*Channichthyidae*), oxygen is carried from the lungs to the tissues by the RBC, which makes this cell an integral player in almost all physiological processes. Studying RBC transport in capillaries is particularly important, because even though the cardiovascular system is responsible for the distribution of blood, it is the microcapillary network that ensures oxygen and nutrient delivery to the tissues and organs. Approximately 80% of the overall pressure drop in systemic circulation occurs in the microcirculation. Blood flow in microcirculation is dependent on the intrinsic RBC biomechanical properties, namely deformability and adhesiveness. Tight squeeze of the RBC as it passes through the narrow capillaries exposes the entire circumference of the cell to the vascular wall adhesion molecules, which renders the microvessels more susceptible to detrimental effects of RBC adhesion, compared to large vessels. RBC deformability and adhesion are compromised in many disease states, resulting in blockages of blood flow in microcapillary networks.

RBC's reduced deformability, increased adhesion, and PS exposure have been associated with microcirculatory impairment in many diseases, including anemias, sepsis, malaria, lupus, heavy metal exposure, blood transfusion complications, diabetes, cancer, kidney diseases, cardiovascular diseases, obesity, and neurological disorders. These diseases affect hundreds of millions of people globally with a socioeconomic burden of hundreds of billions of dollars annually. For example, in sickle anemia, RBC adhesion has been associated with blood flow blockage, disease severity, and organ damage. RBC is an important target of mercury and even low levels of mercury can induce PS exposure, and adhesion in circulation. Lead exposure has been shown to damage RBC membrane, result in reduced deformability, and reduced membrane elasticity. Blood storage has been shown to induce oxidative stress, osmotic stress, PS exposure, and reduced deformability in RBCs, which have been associated with organ failure. A fundamental and unified understanding of RBC's adhesion affinity, deformability, and PS exposure will potentially lead to novel monitoring and treatment strategies for microcirculatory disorders, which may improve the lives of millions of people.

Hemoglobin Biochip (HemeChip)

Some embodiments described herein relate to a electrophoresis biochip and system that accurately identifies and quantitates hemoglobin protein from a subject. The electrophoresis biochip can be used in a system for detecting hemoglobin disorders, such as hemoglobinopathy, thalassemia, sickle cell disease, sickle cell anemia, congenital dysterythropoietic anemia, and mild chronic anemia.

Referring to Figs. 1 and 2, the electrophoresis biochip can include a housing having first and second buffer ports, a sample loading port, and first and second electrodes. The housing also includes a microchannel that extends from a first end to a second end of the housing. The microchannel contains cellulose acetate paper that is at least partially saturated with an alkaline buffer solution. The first buffer port and the second buffer extend, respectively, through the first end and second of the housing to the microchannel and cellulose acetate paper. The first buffer port and the second buffer port are capable of receiving the alkaline buffer solution that at least partially saturates the cellulose acetate paper. The sample loading port can receive a blood sample and extends through the first end of the housing to the microchannel and cellulose acetate paper. The first electrode and the second electrode can generate an electric field across the cellulose acetate paper effective to promote migration of hemoglobin variants in the blood sample along the cellulose acetate paper. The first electrode and second electrode can extend, respectively, through the first buffer port and the second port to the cellulose acetate paper.

In some embodiments, the housing can include a top cap, a bottom cap, and a channel spacer interposed between the top cap and the bottom cap. The channel spacer can define the channel in the housing. The top cap, bottom cap, and channel spacer can be formed from at least one of glass or plastic.

In some embodiments, the electrophoresis biochip can further include an imaging system for visualizing and quantifying hemoglobin variant migration along the cellulose acetate paper for blood samples introduced into the sample loading port. The housing can include a viewing area for visualizing the cellulose acetate paper and hemoglobin variant migration.

The first electrode and the second electrode can be connected to a power supply. The power supply can generate an electric field of about 1V to about 400V. In some embodiments, the voltage applied to the biochip by the electrodes does not exceed 250V.

The biochip can be microengineered and be capable of processing a small blood volume (*e.g.*, for example, a fingerprick volume or a heelprick volume).

In other embodiments, the blood sample introduced into the sample loading port can be less than 10 microliters. The buffer solution can include alkaline tris/Borate/EDTA buffer solution. The first electrode and the second electrode can include graphite or carbon electrodes.

In other embodiments, the imaging system can include a mobile phone imaging system to visualize and quantify hemoglobin variant migration. For example, as shown in Fig. 7, the mobile phone imaging system can include a mobile telephone that is used to image hemoglobin variant migration and a software application that recognizes and quantifies the hemoglobin band types and thicknesses to make a diagnostic decision. The hemoglobin band types can include hemoglobin types C/A, S, F, and A0.

In other embodiments, a mobile imaging and quantification algorithm can be integrated into the biochip device. The algorithm can achieve reliable and repeatable test results for data collected in all resource settings of the biochip device.

Imaging of the electrophoresis biochip and data analysis may be performed using a mobile application to enhance reliability and reproducibility of blood analyses. Wang et al., "Micro-a-fluidics ELISA for rapid CD4 cell count at the point-of-care" Scientific Reports 4:3796 (2014). Briefly, an image processing algorithm can initially recognize a microfluidic biochip channel by using exemplary sample ports as position markers. Then, red (R) pixel values may be extracted from a color image and normalized with respect to background. Red pixel intensity histograms may be plotted automatically along the channel, thereby determining the positions of highest intensity. (Fig. 7) The application segments, counts, and quantifies the bands that correspond to different Hb types, and hence different Hb disorders on HemeChip. For example, HbA and/or HbS positions can be determined for each sample using histogram plots, and the results displayed on a screen. Graphical user interface includes essential features, including fiducial markers that guide the user to properly align the camera field-of-view. The application can input date, location, and a unique patient identifier.

In some embodiments, the electrophoresis biochip can be used to diagnose whether the subject has hemoglobin genotypes HbAA, HbSS, HbSA, HbSC, or HbA2. In other embodiments, the electrophoresis biochip can be used to diagnose whether the subject has or an increased risk of sickle cell disease.

In some embodiments, the electrophoresis biochip can be used in a method where a blood sample from a subject is introduced into the sample loading port. The blood sample includes at least one blood cell. Hemoglobin bands formed the cellulose acetate paper are then imaged with the imaging system to determine hemoglobin phenotype for the subject. The hemoglobin phenotype can selected from the group consisting of HbAA, HbSA, HbSS, HbSC, and HbA2.

In some embodiment, an HbAA hemoglobin phenotype diagnoses the subject as normal, an HbSA hemoglobin phenotype diagnoses the subject as having a sickle cell trait, an HbSS hemoglobin phenotype diagnoses the subject as having a sickle cell disease, an HbSC hemoglobin phenotype diagnoses the subject as having a hemoglobin SC disease, and an HbA2 hemoglobin phenotype diagnoses the subject as having thalassemia.

In other embodiments, the electrophoresis biochip can be used in a method for hemoglobin screening capable of identifying an early SCD diagnosis. In one embodiment, the early diagnosis is performed on a newborn infant. In another embodiment, the early diagnosis is performed on an adult. In still other embodiments, the method differentiates between healthy individuals, sickle cell trait carriers, and SCD patients.

In some embodiments, the electrophoresis biochip comprises biomedical grade poly methyl methacrylate (PMMA, McMaster-Carr) substrates and a double sided adhesive film (DSA)(3M Company), which have been shown to be biocompatible and non-cytotoxic in biomedical and clinical applications. Biochips may be fabricated using a micromachining platform (*e.g.*, X-660 Laser, Universal Laser Systems) to create a variety of structures including, but not limited to, inlet ports, outlet ports, sample ports, microfluidic channels, reaction chambers, and/or electrophoresis channels. (Fig. 1A) Microfluidic channel dimensions may be controlled to within 10 micrometers. In other embodiments, the microfluidic biochip system allows rapid manual assembly and is disposable (*e.g.*, for example, a single use biochip) to prevent potential cross-contamination between patients.

One advantage of an electrophoresis biochip design is that it is suitable for mass-production which provides efficiency in point-of-care technologies. The electrophoresis biochip can provide a low cost screen test for SCD (and other hemoglobin disorders), which takes 10 minutes to run. It is mobile and easy-to-use; it can be performed by anyone after a short (30 minute) training. The electrophoresis biochip described herein can integrate with a mobile device (*e.g.*, IPhone, IPod) to produce objective and quantitative results. If necessary, biochips and/or their components may be sterilized (*e.g.*, by UV light) and assembled in sterile laminar flow hood. Sterile biomedical grade silicon tubing (Tygon Biopharm Plus) may be integrated to the biochips and biochips may be sealed to prevent any leakage. Further, tubing allows simple connection to other platforms, such as *in vitro* culture systems for additional analyses if needed.

Microfluidic Biochip

Other embodiments described herein relate to microfluidic biochip system and an analytic method for simultaneous interrogation of blood cell deformability and adhesion to a microvasculature-mimicking surface at a single cell level. In some embodiments, the microfluidic biochip device or system can quantitate membrane, cellular and adhesive properties of red blood cells and white blood cells of a subject to monitor disease severity, upcoming pain crisis, treatment response, and treatment effectiveness in a clinically meaningful way. In one embodiment, the blood cells are derived from whole blood of patients being screened and/or monitored for sickle cell disease (SCD) progression.

The microfluidic biochip device includes a housing and at least one microchannel that defines at least one cell adhesion region in the housing. The at least one cell adhesion region is coated with at least one bioaffinity ligand that adheres a cell of interest when a fluid containing cells is passed through the at least one microchannel. The bioaffinity ligands can include at least one of fibronectin, laminin, selectin, von Willebrands' Factor, thrombomodulin or a C146 antibody. The device also includes an imaging system that measures the quantity of cells adhered to the at least one bioaffinity ligand within the at least one microchannel when the fluid is passed through the channels.

Figs. 8 and 9 illustrate a microfluidic biochip in accordance with one embodiment. The microfluidic biochip includes a housing defining a plurality of channels that include cell adherence regions. Each channel connects to an inlet port at one end and an outlet port at another end. Although Fig. 8 depicts only three channels, the microfluidic device can include more or less than three channels. The diameter of channels should be large enough to prevent clogging of the channels when blood is passed through the channels.

Fig. 13 shows the microfluidic biochip can include a multilayer structure formed of a base layer, an intermediate layer, and a cover layer. The channels are formed in the intermediate layer; the inlet port and outlet port are formed in the cover. A first end of each channel is aligned with its corresponding inlet port and a second end of each channel is aligned with its corresponding outlet port, thus creating a flow channel from an inlet port to the corresponding outlet port via the channel. In some embodiments, the channels extend slightly beyond their respective inlet and outlet ports. The channels are sized to accept, *e.g.,* microliter or milliliter volumes of blood or a solution containing cells to be adhered or captured. The channels may also be further sized and shaped to affect adherence or capturing of the cells.

The base layer provides structural support to the cell adherence region and is formed of a sufficiently rigid material, such as polymethylmethacrylate) (PMMA) or glass in a suitable thickness, such as about 0.1 mm to about 2 mm, for example about 1.6 mm, which is determined by manufacturing and assembly restrictions. The cover layer contains the inlet and outlet port that are used to feed the blood in/out of the channels. The cover layer thickness can be about 1 mm to about 10 mm, for example, about 3.6 mm, and is determined by the integration and assembly requirements. The inlet and outlet port diameters can be about 0.3 mm to about 3 mm, for example about 1mm, where the lower limit is determined by the manufacturing restrictions and the upper limit is determined by the flow conditions of blood.

In some embodiments, a laser cutter can be used as needed to cut a larger piece of PMMA into a desired size for the microfluidic chip and to cut holes for the inlet ports and outlet ports.

The intermediate layer can be formed of a material that adheres to both base and cover layers. The channels can be formed, for example, by laser cutting polygons, such as rectangular sections, in the intermediate layer, which is itself laser cut to the desired size (*e.g.,* the size of the base layer). The height of the channels can be determined by the thickness of the intermediate layer, which is discussed in greater detail below.

In some embodiments, the device geometry and dimensions are determined to accommodate a uniform laminar flow condition for blood, which determines capture efficiency and the flow rate. The channel width can be about 1 mm to about 15 mm, for example, about 3.5 mm, where the minimum width is determined by inlet and outlet port diameters and upper limit for the channel width is determined by the flow characteristics of blood in a confined channel. The channel length can be about 4 mm to about 100 mm, for example about 27 mm. The lower channel length dimension is determined by the flow characteristics of blood in a confined channel and the upper limit is determined by cell capture efficiency. The channel height can be about 10 $\mu$m to about 500 $\mu$m, for example, about 50 $\mu$m, which is determined by the fluid mechanics laws and constraints and flow characteristics of blood in a confined channel.

After the channels are cut into the intermediate layer, the intermediate layer is adhered to the base layer. The cover layer, which can have the same lateral dimensions as the base layer and the intermediate layer, can be adhered onto the exposed side of the intermediate layer, thereby enclosing the channels. In the embodiment depicted in Fig. 13, the microfluidic biochip device is oriented such that the cover layer is on the top. In other embodiments, the cell isolation device may be oriented such that the cover layer is on the bottom.

The cell adherence regions of the microfluidic biochip device can include a surface on which is provided a layer or coating of the at least one bioaffinity ligand. The bioaffinity ligand can include at least one of fibronectin, laminin, selectin, von Willebrands' Factor, thrombomodulin or a C146 antibody. The bioaffinity ligand can be adhered to, functionalized, or chemically functionalized to the cell adhesion region of each channel.

The term "functionalized" or "chemically functionalized," as used herein, means addition of functional groups onto the surface of a material by chemical reaction(s). As will be readily appreciated by a person skilled in the art, functionalization can be employed for surface modification of materials in order to achieve desired surface properties, such as biocompatibility, wettability, and so on. Similarly, the term "biofunctionalization," "biofunctionalized," or the like, as used herein, means modification of the surface of a material so that it has desired biological function, which will he readily appreciated by a person of skill in the related art, such as bioengineering.

The bioaffinity ligands may be functionalized to the cell adhesion region covalently or non-covalently. A linker can be used to provide covalent attachment of a bioaffinity ligand to the cell adhesion region. The linker can be a linker that can be used to link a variety of entities.

In some embodiments, the linker may be a homo-bifunctional linker or a hetero-bifunctional linker, depending upon the nature of the molecules to be conjugated. Homo-bifunctional linkers have two identical reactive groups. Hetero-bifunctional linkers have two different reactive groups. Various types of commercially available linkers are reactive with one or more of the following groups: primary amines, secondary amines, sulphydryls, carboxyls, carbonyls and carbohydrates. Examples of amine-specific linkers are bis(sulfosuccinimidyl) suberate, bis[2-(succinimidooxycarbonyloxy)ethyl]sulfone, disuccinimidyl suberate, disuccinimidyl tartarate, dimethyl adipimate 2HCl, dimethyl pimelimidate 2HCl, dimethyl suberimidate HCl, ethylene glycolbis-[succinimidyl-[succinate]], dithiolbis(succinimidyl propionate), and 3,3'-dithiobis(sulfosuccinimidylpropionate). Linkers reactive with sulfhydryl groups include bismaleimidohexane, 1,4-di-[3'-(2'-pyridyldithio)-propionamido)]butane, 1-[p-azidosalicylamido]-4-[iodoacetamido]butane, and N-[4-(p-azidosalicylamido)butyl]-3'-[2'-pyridyldithio]propionamide. Linkers preferentially reactive with carbohydrates include azidobenzoyl hydrazine. Linkers preferentially reactive with carboxyl groups include 4-[p-azidosalicylamido]butylamine.

Heterobifunctional linkers that react with amines and sulfhydryls include N-succinimidyl-3-[2-pyridyldithio]propionate, succinimidyl[4-iodoacetyl]aminobenzoate, succinimidyl 4- [N-maleimidomethyl]cyclohexane-1-carboxylate, m-maleimidobenzoyl-N-hydroxysuccinimide ester, sulfosuccinimidyl 6-[3-[2-pyridyldithio]propionamido]hexanoate, and sulfosuccinimidyl 4-[N-maleimidomethyl]cyclohexane-1-carboxylate. Heterobifunctional linkers that react with carboxyl and amine groups include l-ethyl-3-[3-dimethylaminopropyl]-carbodiimide hydrochloride. Heterobifunctional linkers that react with carbohydrates and sulfhydryls include 4-[N-maleimidomethyl]-cyclohexane-1-carboxylhydrazide HC1, 4-(4-N-maleimidophenyl)-butyric acid hydrazide.2HCl, and 3-[2-pyridyldithio]propionyl hydrazide.

Alternatively, the bioaffinity ligands may be non-covalently coated onto the cell adhesion region. Non-covalent deposition of the bioaffinity ligand to the cell adhesion region may involve the use of a polymer matrix. The polymer may be naturally occurring or non-naturally occurring and may be of any type including but not limited to nucleic acid (*e.g.,* DNA, RNA, PNA, LNA, and the like, or mimics, derivatives, or combinations thereof), amino acid (*e.g.,* peptides, proteins (native or denatured), and the like, or mimics, derivatives, or combinations thereof, lipids, polysaccharides, and functionalized block copolymers. The receptor may be adsorbed onto and/or entrapped within the polymer matrix.

Alternatively, the bioaffinity ligand may be covalently conjugated or crosslinked to the polymer (*e.g.,* it may be "grafted" onto a functionalized polymer).

An example of a suitable peptide polymer is poly-lysine (*e.g.*, poly-L-lysine). Examples of other polymers include block copolymers that comprise polyethylene glycol (PEG), polyamides, polycarbonates, polyalkylenes, polyalkylene glycols, polyalkylene oxides, polyalkylene terepthalates, polyvinyl alcohols, polyvinyl ethers, polyvinyl esters, polyvinyl halides, polyvinylpyrrolidone, polyglycolides, polysiloxanes, polyurethanes, alkyl cellulose, hydroxyalkyl celluloses, cellulose ethers, cellulose esters, nitrocelluloses, polymers of acrylic and methacrylic esters, methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxylethyl cellulose, cellulose triacetate, cellulose sulphate sodium salt, poly(methyl methacrylate), poly(ethyl methacrylate), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), polyethylene, polypropylene, poly(ethylene glycol), poly(ethylene oxide), poly(ethylene terephthalate), poly(vinyl alcohols), polyvinyl acetate, polyvinyl chloride, polystyrene, polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate), poly(lactide-glycolide), copolyoxalates, polycaprolactones, polyesteramides, polyorthoesters, polyhydroxybutyric acid, polyanhydrides, poly(styrene-b-isobutylene-b-styrene) (S1BS) block copolymer, ethylene vinyl acetate, poly(meth)acrylic acid, polymers of lactic acid and glycolic acid, polyanhydrides, poly(ortho)esters, polyurethanes, poly(butic acid), poly(valeric acid), and poly(lactide-cocaprolactone), and natural polymers such as alginate and other polysaccharides including dextran and cellulose, collagen, albumin and other hydrophilic proteins, zein and other prolamines and hydrophobic proteins, copolymers and mixtures thereof, and chemical derivatives thereof including substitutions and/or additions of chemical groups, for example, alkyl, alkylene, hydroxylations, oxidations, and other modifications routinely made by those skilled in the art.

In some embodiments, each microchannel can include separate cell adhesion regions coated with at least one bioaffinity ligand. At least two or at least three of the microchannels can include different bioaffinity ligands. In other embodiments, the plurality of microchannels can include the same bioaffinity ligands.

In still other embodiments, at least one microchannel can include at least two different bioaffinity ligands coated on the cell adhesion region of the microchannel. The different bioffinity ligands can be located at different positions within the cell adhesion region of at least one microchannel. For example, at least one of the laminin, the selectin, the von Willebrands' Factor, the thrombomodulin and the C146 antibody are localized at different positions along the at least one microchannel.

In some embodiments, the imaging system can be a lens based imaging system or a lensless imaging system. For example, the lensless imaging system can be a charged coupled device sensor and a light emitting diode. In some embodiments, a mobile imaging and quantification algorithm can be integrated into the biochip device. The algorithm can achieve reliable and repeatable test results for data collected in all resource settings of the biochip device.

In other embodiments, the cells can be blood cells obtained from the subject and the imaging system can quantify the adhered cells in each respective channel to monitor heath of a subject from which the cells are obtained. In other embodiments, the imaging system can quantify the adhered cells in each respective channel to monitor the progression of a disease, such as sickle cell disease, of a subject from which the cells are obtained. In still other embodiments, the imaging system can quantify the adhered cells in each channel to measure the efficacy of a therapeutic treatment administered to a subject from which the cells are obtained.

In some embodiments, the microfluidic biochip device can be used in a method where a blood sample comprising at least one blood cell from a subject is introduced into the microchannel and the quantity of cells adhered to the at least one bioaffinity ligand within the at least one microchannel is imaged. The biochip device can quantitate membrane, cellular and adhesive properties of red blood cells and white blood cells of a subject to monitor disease severity, upcoming pain crisis, treatment response, and treatment effectiveness in a clinically meaningful way.

It can be expected that a microfluidic biochip platform disclosed herein is applicable to the study of single cell heterogeneity of adherent cells within subjects in larger clinically diverse populations and may provide important insights into complex disease phenotypes other than SCD. For example, abnormal RBC adhesion to microvascular surfaces has been implicated in other multi-system diseases, such as β-thalassemia, diabetes mellitus, hereditary spherocytosis, polycythemia vera, and malaria. Connes et al., British journal of haematology 165(4):564-572 (2014); Colin et al., Current opinion in hematology 21(3):186-192 (2014); and Cooke et al., Parasitology 107:359-368 (1993).

Sickled RBC adherence to blood vessel walls has been shown to take place in post-capillary venules. In one embodiment, the present invention contemplates a microfluidic SCD biochip comprising at least one microchannel having a width of approximately 60 $\mu$m and fluid flow velocities within a range of approximately 1-10 mm/sec, that have been reported for post-capillary venules. Kaul et al., Microcirculation 16(1):97-111 (2009); Kaul et al., Proceedings of the National Academy of Sciences of the United States of America 86(9):3356-3360 (1989); Lipowsky, H. H., Microcirculation 12(1):5-15 (2005); and Turitto, V. T., Progress in hemostasis and thrombosis 6:139-177 (1982).

Studies using similar flow conditions, based on extrapolations from physiological states in SCD, have been reported in the literature. Montes et al., American journal of hematology 70(3):216-227 (2002);.Kasschau et al., Blood 87(2):771-780 (1996); Lei et al., Proceedings of the National Academy of Sciences of the United States of America 110(28):11326-11330 (2013). However, no study analyzes HbS-containing RBC adhesion and deformability using whole blood at the microvasculature scale of ~ 60 $\mu$m. The data presented herein demonstrate heterogeneity in HbS-containing RBC adhesion and deformability measured at a single cell level in SCD blood samples examined in microfluidic channels mimicking a microvasculature.

In one embodiment, the microfluidic biochip can be used in an SCD testing method utilizing pathophysiologic correlates, including but not limited to, analyses of adhesion and membrane properties in HbSS and HbSC, at baseline and during vaso-occlusive crises, with treatment, and in the presence of end-organ damage. The SCD testing method can be completed in less than ten minutes. In some embodiments, the SCD testing method provides a highly specific analyses of CMA properties in RBCs, WBCs, circulating hematopoietic precursor cells and circulating endothelial cells. In one embodiment, the SCD testing method is performed using a portable, high efficiency, microfluidic biochip and a miniscule blood sample (<15 $\mu$l). The SCD testing method can provide a sophisticated and clinically relevant strategy with which patient blood samples may be serially examined for cellular/membrane/adhesive properties during SCD disease progression.

In some embodiments, the biochip can accurately quantitate cellular interactions and membrane properties using a single drop of blood. The biochip and method may validate insights about mechanisms of disease in SCD and may reveal correlations between disease heterogeneity and acute and/or chronic SCD complications.

The microfluidic biochip can also evaluate membrane and cellular abnormalities by interrogating a number of recognized abnormalities in a range of clinical phenotypes. To date, these phenotypes are discussed in various correlative SCD studies ranging between clinical reports, testing results, interventions, and/or chart reviews. Lorch et al., "An elevated estimated pulmonary arterial systolic pressure, whenever measured, is associated with excess mortality in adults with sickle cell disease" Acta Haematol 125:225-229 (2011); Powars et al., "Outcome of sickle cell anemia: a 4-decade observational study of 1056 patients" Medicine (Baltimore) 84:363-376 (2005); Nouraie et al., "The relationship between the severity of hemolysis, clinical manifestations and risk of death in 415 patients with sickle cell anemia in the US and Europe" Haematologica 98:464-472 (2013); Platt et al., "Mortality in sickle cell disease. Life expectancy and risk factors for early death" N Engl J Med 330:1639-1644 (1994); Ohene-Frempong et al., "Cerebrovascular accidents in sickle cell disease: rates and risk factors" Blood 91:288-294 (1998); West et al., "Laboratory profile of sickle cell disease: a cross-sectional analysis. The Cooperative Study of Sickle Cell Disease" J Clin Epidemiol 45:893- 909 (1992); Charache et al., "Effect of hydroxyurea on the frequency of painful crises in sickle cell anemia. Investigators of the Multicenter Study of Hydroxyurea in Sickle Cell Anemia" N Engl J Med 332:1317-1322 (1995). Lettre et al., "DNA polymorphisms at the BCL11A, HBS1L-MYB, and beta-globin loci associate with fetal hemoglobin levels and pain crises in sickle cell disease" Proc Natl Acad Sci U S A 105:11869-11874 (2008); Milton et a1., "Genetic determinants of haemolysis in sickle cell anaemia" Br J Haematol 161:270-278 (2013); Bae et a1., "Meta-analysis of 2040 sickle cell anemia patients: BCL11A and HBS1L-MYB are the major modifiers of HbF in African Americans" Blood 120:1961-1962 (2012); and Milton et a1., "A genome-wide association study of total bilirubin and cholelithiasis risk in sickle cell anemia" PLoS One 7:e34741 (2012).

It is generally known that among common blood disorders, SCD is unusually detectable by an examination of cell membrane properties and cellular activation. For example, many observations about membrane and cellular abnormalities, and their relationship to clinical complications in SCD, have been made since the 1980's. However, most pathophysiologic studies have been undertaken in modest numbers of subjects at a single time point at a single institution. Further, it has not been feasible for more than one analysis to be performed on a single patient sample, *e.g.,* simultaneous evaluations of membrane properties, inflammatory cell activation, and circulating endothelial cell number. Often, promising 'cutting-edge' or biologically illuminating correlative tests are too expensive, complex, or difficult to 'export' to other centers. These disadvantages of the present methods have prevented a widespread access to longitudinal examination of pathophysiologic endpoints, such as intercellular adhesion of RBCs, WBCs, and endothelium (and subendothelium).

Further, third world countries carry the burden of SCD. Therefore, an inexpensive and simple point-of-care device to provide discriminants and diagnostics of disease activity could be extremely valuable tools world-wide. Yang et a1., A simple, rapid, low-cost diagnostic test for sickle cell disease" Lab on a Chip 13:1464-1467 (2013). Yang et al. describes a diagnostic test for sickle cell disease (SCD) using paper chromatography. Here, the presently disclosed improvements in SCD monitoring and testing broaden pathophysiologic endpoint availability through a microfluidic SCD biochip technology. In one embodiment, the microfluidic SCD biochip performs a testing method measuring cellular adhesion. In one embodiment, the SCD biochip measures red blood cell (RBC) binding to laminin (LN). In one embodiment, the SCD biochip measures white blood cell binding to selectins. In one embodiment, the SCD biochip measures cell activation including, but not limited to white blood cell activation, endothelial cell activation and/or hematopoietic precursor cell activation.

The present embodiments have advantages because existing conventional methods cannot assess longitudinal and large-scale SCD clinical correlations with CMA properties. In one embodiment, the present invention contemplates a method for using an SCD biochip for examining cellular properties and interactions. In one embodiment, these cellular properties and interactions include, but are not limited to, red blood cell (RBC) cellular and adhesive properties, white blood cell (WBC) cellular and adhesive properties, circulating endothelial characteristics and hematopoietic precursor cell characteristics. In one embodiment, the present invention contemplates a method for correlating SCD biochip function in heterogeneous SCD populations, including but not limited to, HbSS and HbSC over a range of ages, and in those with acute and chronic complications and compared with normal HbAA controls.

While the present data validates a focused panel of SCD cellular properties, one skilled in the art would recognize that this technology may be more broadly applicable to additional pathologic cellular interactions in SCD. Fig. 8. Consequently, an SCD biochip allows a widespread clinical application of pathophysiologic disease correlates, heretofore not feasible. This approach further may yield a uniquely valuable research material, such as precisely isolated cellular populations that have been implicated in SCD pathogenesis (e.g., endothelial cells and inflammatory cells). Furthermore, some embodiments of an SCD biochip can explore endpoints for use in future therapeutic trials, applicable in the U.S. and in resource-limited settings worldwide where SCD is most prevalent, such as South America and Africa.

Microfluidic biochip technology described herein can investigate surface characteristics that are typically measured with conventional techniques, such as fluorescent activated cell sorting (FACS), immunohistochemistry, or microscopic imaging methods. In FACS, cells of interest are isolated, extensively processed, incubated with a fluorescent-labeled antibody raised against a cellular protein (e.g., integrin, receptor, adhesion molecule), and sorted by optical recognition. In one embodiment, the present invention contemplates a microfluidic biochip (e.g., a microfluidic SCD biochip) comprising an interrogating antibody. In one embodiment, the antibody coats a microchannel surface. In one embodiment, the antibody captures a cell population of interest, without preprocessing, incubation, or in vitro manipulation.

In one embodiment, the microfluidic biochip quantitates adherence of cellular populations to subcellular components including, but not limited to lignin, adhesion molecules, or selectins. In some embodiments, the microfluidic biochip can capture and quantifies RBCs, WBCs, and circulating endothelial and hematopoietic precursor cells based on membrane properties and adhesion. For example, a microfluidic biochip comprises a plurality of microchannels that are functionalized with lignin, selectins, avidin and/or biotinylated antibodies to BCMA/LU, CD11b, CD34, and/or CD146.

A simple test for adhesion would allow exploration of its role in chronic complications in SCD, in addition to during crisis. Selectins may be tested using microfluidic biochips as an adhesive surface, in place of cultured endothelial cells. Endothelial selectins are believed to mediate leukocyte adhesion and rolling on the endothelial surface. For example, in experimental models of SCD, P-selectin is widely expressed on vascular endothelium and endothelial E-selectin is important for vascular occlusion. Wood et al., "Differential expression of E- and P-selectin in the microvasculature of sickle cell transgenic mice" Microcirculation 11:377-385 (2004); Wood et al., "Endothelial cell P-selectin mediates a proinflammatory and prothrombogenic phenotype in cerebral venules of sickle cell transgenic mice" American Journal Of Physiology Heart And Circulatory Physiology 286:H1608-1614 (2004); and Hidalgo et al., "Heterotypic interactions enabled by polarized neutrophil microdomains mediate thromboinflammatory injury" Nat Med 15:384-391 (2009). Neutrophils in SCD show greater adherence to endothelium, via neutrophilic MAC-1, LFA-1, and, unique to SCD, VLA-490, which may be altered with effective therapy. Almeida et al., "Hydroxyurea and a cGMP-amplifying agent have immediate benefits on acute vaso-occlusive events in sickle cell disease mice" Blood 120:2879-2888 (2012).

In one embodiment, the present invention contemplates a microfluidic SCD biochip comprising at least one microchannel with at least one immobilized P-selectin and/or E-selectin adhesion molecules. It is expected that SCD samples show greater WBC adherence to selectins, compared with HbAA controls. Examination of SCD samples, at baseline and with crisis, may evaluate changes with disease activity. For example, MAC-1, LFA-1, and VLA-4 expression may be measured by FACS on selectin-captured blood WBCs, as compared with unmanipulated WBCs on an SCB microfluidic biochip from the same sample.

It is possible that immobilized selectins may interact with RBCs. If this hinders analysis of WBC interactions, RBCs may be lysed prior to analysis. Matsui et al., "P-selectin mediates the adhesion of sickle erythrocytes to the endothelium" Blood 98:1955-1962 (2001). RBC adherence may be variable between patients and therefore informative, and can be quantified if so. Of note, conventional *in vitro* measures of WBC adherence entail endothelial cell culture which is avoided by use of a microfluidic biochip system.

Monocytes are recognized as major inflammatory mediators of endothelial activation in SCD. Belcher et al., "Activated monocytes in sickle cell disease: potential role in the activation of vascular endothelium and vaso-occlusion" Blood 96:2451-2459 (2000); and Perelman et al., "Placenta growth factor activates monocytes and correlates with sickle cell disease severity" Blood 102:1506-1514 (2003). In one embodiment, a SCD microfluidic biochip comprising a CD11b isolates an activated WBC. In one embodiment, the activated WBC is a monocyte.

In other embodiments, the microfluidic biochip can include a microchannel coated with CD146 antibodies to quantitate mature circulating endothelial cells. In another embodiment, the present invention contemplates a microfluidic SCD biochip comprising vWF or thrombomodulin to quantitate CECs by image analysis. Lin et al., "Origins of circulating endothelial cells and endothelial outgrowth from blood" J Clin Invest 105:71-77 (2000). Although isolation of rare CECs is technically challenging, these cells have been identified by FACS in unmanipulated blood using a CD146 marker. Samsel et al., "Imaging flow cytometry for morphologic and phenotypic characterization of rare circulating endothelial cells" Cytometry Part B, Clinical Cytometry (2013).

Biophysical probing of individual cells in a microfluidic biochip described herein necessitates accurate control, measurement, and estimation of flow velocities in close vicinity to the adhered cells. These measured and estimated values allow theoretical calculations about flow in the microfluidic channels. Validation of the accuracy of these predicted flow velocities in comparison with measured local flow velocities may be performed through particle image tracking around the adhered cells and using the non-adhered flowing cells as free flowing particles with which to measure the local flow velocities. (Fig. 11A) When free flowing particles, such as cells, are imaged at relatively long camera exposure times, they appear as straight lines due to motion blur, which is also known as streaking. The length of these streaks is proportional to the flowing particle velocity. (Fig. 11A). Local flow velocities ($v_p$) for flowing cells are determined by dividing the streaking line length ($L_p$) minus average cell size ($d_p$) to camera exposure duration ($t_p$) using Equation 1:

$$V_p = (L_p - d_p)/t_p \qquad (1).$$

Then, a correlation is analyzed between the measured local flow velocity and the predicted mean flow velocity ($v_m$) determined by the volumetric flow rate ($Q$) and the dimensions of the microchannels (width: $w_c$, and height: $h_c$) using Equation 2:

$$v_m = Q/(w_c \: x \: h_c) \qquad (2).$$

The data show that measured local flow velocities display a significant correlation (Pearson correlation coefficient of 0.94, p<0.001, n=9, R2=0.88) with the predicted flow velocities. (Fig. 11B) Hence, a mean theoretical flow velocities was used in determining the shear stress and drag force levels for detachment of HbA and HbS-containing RBCs. (Figs. 11C, 11D and 11E).

The relative positions of adhered RBCs and flow velocities were determined for the adhered RBCs, as well as shear stresses and drag forces acting on the cells at detachment. Adhered RBCs were positioned in the mid-region of the microchannels where the flow velocity was uniform across the channel. The data show that either: i) up to 4.3 times greater flow velocity (Fig. 11C); ii) 4.3 times greater shear stress (Fig. 11D); and iii) 4.1 times greater drag force (Fig. 11E) was required to detach non-deformable HbS-containing RBCs as compared to HbS-containing deformable RBCs (p<0.05, one way ANOVA with Fisher's post-hoc test). In contrast, HbA-containing RBCs and deformable HbS-containing RBCs did not differ in terms of flow velocity, shear stress, and drag force at detachment (p>0.05, one way ANOVA with Fisher's post-hoc test). These results confirm that HbS-containing RBCs are heterogeneous in terms of their adhesion strength to FN.

The motion of adhered RBCs at flow initiation was evaluated using consecutive high resolution microscopic images taken over 0.28 seconds, in order to analyze sites of adhesion in HbA, HbS deformable, and HbS non-deformable RBCs. (Figs. 12A-L). The data demonstrate that HbA and HbS deformable cells display rotational motion in response to fluid flow direction, indicating a single adhesion pivot point. (Figs. 12D and 12H). On the other hand, HbS non-deformable cells do not display a rotational motion in response to fluid flow direction, implying multiple adhesion sites. (Fig. 12L). Importantly, these observations suggest that the higher adhesion strength of HbS non-deformable cells may be due to a greater number of adhesion sites.

Examples

Example 1

The HemeChip technology offers a novel and innovative solution to the challenge faced by clinical facilities in diagnosing SCD. In as little as ten minutes, HemeChip uses cellulose acetate electrophoresis to separate less than five microliters of blood into bands displaying the basic types of hemoglobin: A, A2, S, C and F. The cost of running and making each chip is less than $2.00, and the Hemechip system is easy to use and transport. Moreover, the chip's simple design is easy to mass produce. Hemechip technology provides an accurate, efficient, rapid, and inexpensive way to diagnose SCD.

Materials and Methods

HemeChip Materials

Poly (methyl methacrylate) (PMMA) sheets of 1.5 mm thickness were purchased from McMaster-Carr (Elmhurst, IL), and 1/32" thick PMMA sheets were purchased from ePlastics (San Diego, CA). 3M optically clear double sided adhesive (DSA) (Type 8142) was purchased from iTapeStore (Scotch Plains, NJ). A 1× Tris/Borate/EDTA (TBE) buffer solution (pH 8.3) was made from a 10× TBE Buffer solution (Invitrogen™, Carlsbad, CA), diluted with deionized water (MilliQ Academic, Billerica, MA). Cellulose acetate membranes were purchased from Apacor and distributed by VWR International LLC (Radnor, PA). A 300V power supply was purchased from VWR International LLC (Model 302, Radnor, PA). Ponceau S Stain, Hemoglobin AFSC control, and Super Z micro-applicator were purchased from Helena Laboratories (Beaumont, TX). Acetic acid glacial was purchased from Fisher Scientific (Waltham, MA). Graphite electrodes (0.9mm) were purchased from Amazon (Seattle, WA). Black 1/8" diameter dots were purchased from Mark-It (Tonawanda, NY).

HemeChip Fabrication and Assembly

The HemeChip is fabricated using PMMA sheets of 1.5 mm and 1/32" thickness. There are 5 layers of PMMA (50 mm × 25 mm), and these layers are assembled using DSA (Fig. 1A). We used the VersaLASER system (Universal Laser Systems Inc., Scottsdale, AZ), a laser micromachining system, for making individual PMMA layers as well as the DSA's to attach the layers. The top and bottom layer of the HemeChip is made of 1/32" thick PMMA sheet, and the rest of the layers are made of 1.5 mm thick PMMA sheet. The DSA has a thickness of 50 μm. The cellulose acetate paper for the experiments is also cut (39 mm × 9 mm) using the VersaLASER system. We have also fabricated a sample loading unit for the HemeChip using a similar manufacturing method with PMMA. The unit was compared to manual stamping by hand through analysis of the cross sectional area and repeatability of the blood sample application.
The CAD designs for the HemeChip were drawn using the CorelDRAW Suite X6 (Corel Corporation, Ottawa, Ontario) and SolidWorks 3D CAD (Waltham, MA) (Fig. 1B). The designs were exported to the interface for VersaLASER system for making those layers. The cutting power for the VersaLASER system was prepped by setting the "vector cutting" from the intensity adjustment for the laser. The PMMA sheets and the DSA were cut with a setting of 45% (min: - 50%, max: 50%) for the "vector cutting". We used a setting of - 40% for cutting the cellulose acetate paper. A non-through cut at the both ends of the plastic back of the cellulose paper was created for bending the paper (Fig. 1C). The bending facilitates the buffer solution and paper contact for the electrophoresis. The non-through cuts were created at 3.0 mm off the ends using a setting of 50% (min: - 50%, max: 50%) of "Raster" setting at the intensity adjustment for the laser.

Blood Preparation

Under Institutional Review Board (IRB) approval, discarded and de-identified patient blood samples were obtained from University Hospital's Hematology and Oncology Division (Cleveland, OH). Blood samples were collected into Vacutainer tubes containing EDTA anticoagulants (BD, Franklin Lakes, NJ). Whole blood samples were stored standing at 10 °C and left to separate into plasma and hematocrit via gravity. The hematocrit of each sample was mixed with deionized water in a 1:5 ratio and placed on an ice block for 15 minutes to lyse the red blood cells. Prepared samples were stored in individual sealed microtubes at 10°C and mixed gently before use. Samples were used and stored up to two weeks from the date received. Alternatively, whole blood samples can be lysed on the chip with a 1% Saponin + TBE buffer solution.

HemeChip Analysis

The cellulose acetate paper was soaked with 40 $\mu$L of 1$\times$ TBE buffer via pipette through the HemeChip's sample loading port until fully saturated by capillary action. Excess buffer was left to dry or redistribute through the paper for 5 minutes. Less than 1 $\mu$L of prepared blood sample was stamped onto the paper using a micro-applicator through the sample loading port. Approximately 200 $\mu$L of 1$\times$ TBE buffer was pipetted into each buffer port. Graphite electrodes (1 inch length) were placed vertically into the buffer ports. The HemeChip was run at a constant voltage of 250V and max current of 5 mA for 8 minutes using a compact power supply. Optionally, Ponceau S stain (25 $\mu$L) was pipetted on to the paper and left to soak for 5 minutes. A 5% acetic acid wash was used to remove the stain until the hemoglobin bands were visible and the paper returned to its original white color. Four black 1/8" diameter dots were placed at each corner for use with the mobile and web-based image processing software.

Image Processing

A Nikon D3200 camera with a 40mm f/2.8G AF-S DX Micro NIKKOR lens (Tokyo, Japan) was used to capture close up pictures of each HemeChip. Images were processed using ImageJ version 1.48 for Windows with no additional plugins. In each image, only the paper was cropped and used for analysis. The Subtract Background feature was used to apply a "rolling ball" algorithm with a radius of 25 pixels to remove smooth continuous background noise from the paper. The Plot Profile, Surface Plot, and Gel Plot tools were used to visualize and quantify the hemoglobin bands. The Plot Profile tool provided the relative pixel intensities along the paper and were used to identify the peaks corresponding to each type of hemoglobin band. The area under each peak was calculated using the Gel Plot tools and represented the relative hemoglobin percentages. The area of each peak was outlined using the valley-to-valley method commonly used in gas chromatography. 3D Surface profiles of hemoglobin bands were obtained with the Surface Plot tool. Band distances were calculated in MATLAB to identify the coordinates of each peak on the profile plot. These were converted from pixels to mm using the HemeChip length-to-pixel ratio obtained from ImageJ. The same procedure was used to quantify the hemoglobin results from the standard benchtop electrophoresis setup.

Data Analysis

The hemoglobin percentages obtained from the HemeChip, HPLC, and benchtop electrophoresis were analyzed and compared using bar and correlation plots. Hemoglobin identification of HbC/A2, HbF, HbS, and HbA0 for the same blood samples that were used for HemeChip experiments was conducted via high-performance liquid chromatography (HPLC) at the Core Laboratory of University Hospitals Case Medical Center, using the Bio-Rad Variant II Instrument (Bio-Rad, Montreal, QC, Canada). Samples were also analyzed with our lab's traditional bench-top electrophoresis setup (Helena Laboratories Model G4063000, Beaumont, TX).

Web-based Image Processing and Quantification

Image processing algorithm: In this example the image processing algorithm is developed using MATLAB software. Briefly, the algorithm first reads the color image, detects the reference points and calibrates the image dimensions of the chip and the channel. Then the algorithm identifies the changes in red, blue and green values for each pixel along a reference line placed in the middle of the channel. This identification leads to detection of the peak values, which correspond to the reddish areas in the channel. Once the reddish areas are determined, the area of each area and the displacement from the start point is calculated. The area and the distance are used to determine the type of hemoglobin disease.

In order to have an image analysis system that is independent from the mobile operating systems, we designed the image-processing module compatible with cloud computing resources. As a result, any mobile device, which has a web browser and Internet connection, can be used to take image from HemeChip, transfer image to cloud computing servers for analysis and receive/display the results on the web browser.

We used MATLAB Compiler SDK™ (Software Development Kit) to produce a .dll file from the MATLAB code to process in .NET framework. The produced .dll file and Bootstrap framework were used to develop Asp.Net project. First, the webpage converts the image of HemeChip into mwArray object and transfers it to .NET library that runs the MATLAB code in the background. Then the output of the function produces another mwArray object and transfers back to the webpage for display. We are planning to increase the time efficiency of the image analysis by removing ASP.NET layer and integrating a fully scalable cloud computing system. In this design image-processing request will be conveyed to queuing service. Queuing service will distribute processing requests to Background Workers in order to execute the requests and scalability will be automatically performed based on the amount of requests. It is also possible to develop a mobile device application instead of running the analysis tool on the web browser. Having an application on the mobile device allows controlling camera features and taking calibrated HemeChip images.

Statistical Analysis

Band traveling distances for different hemoglobin types were statistically assessed (Minitab 16 software, Minitab Inc., State College, PA) using one way Analysis of Variance (ANOVA) test. The correlation and agreement between the measured hemoglobin concentrations for HemeChip and HPLC were evaluated using Pearson-product-moment correlation coefficient and Bland-Altman analysis. Limits of agreement in the Bland-Altman analysis were defined as the meant different $\pm$ 1.96 times the standard deviation of the differences. Statistical significance was set at 95% confidence level for all tests ($p < 0.05$). Receiver-operating curves were utilized to assess differentiation of different hemoglobin phenotypes based on their traveling distances in the HemeChip. Sensitivity was calculated as # true positives / (# true positives + # false negatives) and specificity was calculates as # true negatives / (# true negatives + # false positives). HemeChip data obtained in this study is reported as mean $\pm$ standard deviation. Error bars in the figures represent the standard deviation.

RESULTS

HemeChip Design and Operation

The basis of this technology is hemoglobin electrophoresis and is briefly explained. Hemoglobin types C, A2, S, F, and A0 have net negative charges in a buffer with a pH in the range of 6.5 to 9.0. We used a $1\times$ Tris/Borate/EDTA (TBE) buffer to provide the necessary ions for electrical conductivity at a pH of 8.3. The overall negative net charges of the hemoglobins causes them to travel towards the positive electrode when placed in an electric field. Differences in hemoglobin mobilities allow separation to occur within the sieving medium, cellulose acetate, as shown in Fig. 1C. HbA0 has the highest mobility and travels the furthest. In contrast, HbC/A2 has the lowest mobility and travels the least. In this paper, we group HbC and HbA2 together due to their similar mobilities.
We utilized a micro-engineered design and multiple layer lamination approach in developing the HemeChip. The HemeChip is composed of poly (methyl methacrylate) (PMMA) substrates which encompass the electrodes, buffer ports, and a cellulose acetate paper in which the hemoglobin separation takes place (Fig. 1A). The microchip system allows rapid manual assembly and works with miniscule amounts of blood (Fig. 1D). Finger-stick or heel-stick volume blood samples (20 $\mu$L) were mixed with deionized water for cell lysis and less than 1 $\mu$L was stamped on the cellulose acetate paper in the HemeChip.
Graphite pencil leads were utilized as electrodes to apply constant voltage generated by a power supply (VWR, Model 302). Analysis of a sample takes 8 minutes at a constant 250 V with up to 5 mA. Fig. 2 shows the time lapse of the hemoglobin separation of a patient sample with the sickle cell trait (SCT) on the HemeChip under these conditions. The separation between the HbS and HbA0 bands was visible to the naked eye (Fig. 2A, iii). Staining with Ponceau S is optional for samples with fainter bands as a result of low hematocrit levels. This process can be done on the HemeChip.

HemeChip Quantification

The resulting hemoglobin bands were captured using a digital camera. They were then visualized and quantified using various tools in ImageJ. We plotted the intensity profile of the hemoglobin bands along the length of the cellulose acetate paper (Fig. 3, ii). The origin of each plot corresponds to the center of the sample application point. Each peak represents a hemoglobin band, and the area under each peak represents the relative amount of hemoglobin for that band. We expressed the amount of hemoglobin as a percentage that is relative to that of other hemoglobin bands in the sample (Fig. 3, iv). 3D surface plots were also used to visualize the hemoglobin bands across the cellulose acetate paper (Fig. 3, iii). We have analyzed 12 blood samples with a total of 43 experiments using this method. These samples included the following: 2x HbSS, $1\times$ HbSS HPFH, $1\times$ Cord Blood (High HbF), 3x HbSC, and 5x HbSA. The HemeChip results were benchmarked against HPLC (Bio-Rad VARIANT™ II Hemoglobin Testing System) and traditional benchtop electrophoresis (Helena Laboratories Model G4063000, Beaumont, TX).
Fig. 3A shows the analysis of a SCD patient, homozygous HbSS, with hereditary persistence of fetal hemoblogin (HPFH). HemeChip analysis shows high levels of HbS and low levels HbC/A2 and HbF. These results are consistent with what was obtained through HPLC and benchtop electrophoresis (Fig. 3A, iv). Similarly, Fig. 3B shows the analysis of a patient with the sickle cell trait (SCT), heterozygous HbSA. The HemeChip results show high levels of HbS and some HbA0, agreeing with the standard clinical methods. In contrast, the HemeChip was able to detect SCT for samples with low HbS and high HbA0, which also agreed with the standard methods (Fig. 6, C). Hemoglobin types C/A2, S, F and A0 were successfully identified both visually and quantitatively. A similar analysis for Hemogblin SC disease, HbSC, is shown in Fig. 6. Overall, the HemeChip was able to distinguish between different types of hemoglobin disorders

including homozygous HbSS (SCD), heterozygous HbSA (SCT), and HbSC (hemoglobin SC disease).

HemeChip Diagnostic Efficacy

To assess the diagnostic efficacy of the HemeChip, we compared the hemoglobin percentages to those obtained from HPLC for 12 blood samples with a total of 43 experiments. These samples included the following: 2x HbSS, $1\times$ HbSS HPFH, $1\times$ Cord Blood (High HbF), 3x HbSC, and 5x HbSA. Fig. 4A-E shows the correlation plots for HemeChip vs. HPLC hemoglobin percentages. Each data point represents the mean hemoglobin percentage from a single sample. The solid diagonal lines represent the trend line for each data set. We used the Pearson-product-moment correlation coefficient (PCC) to assess the agreement between the two methods. Hemoglobin types C/A2, S, F, and A0 showed high positive correlation with p<0.001 ($PCC_{HDC/A2}$ - 0.99, $PCC_{HDS}$ - 0.99, $PCC_{HDF}$ - 0,9, $PCC_{HDA0}$ - 0.96). High positive correlation was also observed for all of the hemoglobin types combined (p<0.001, $PCC$ = 0.99) (Fig. 4E).

We also assessed the agreement between the HemeChip and HPLC results using the Bland-Altman plot. Fig. 4F shows strong agreement (SD difference=7%HbS) between estimated (HemeChip) and actual (HPLC) %HbS. The solid line represents the mean difference and the dashed lines represent two standard deviation difference. The majority of the differences between actual and estimated %HbS (95.5%) are within two standard deviations of the mean of the differences. The hemoglobin types are indicated by the colored dots.

Hemoglobin band identification was accomplished by analyzing the travelling distance (mm) of each band from the application point under set conditions (250V, <5mA, 8 min). Fig. 5A compares the travelling distance for each hemoglobin type. The data set consists of 11 different patient blood samples (3x SS, 2x SS HPFH, 2x Cord Blood (high HbF), 2x SC, 2x SA) and 32 experiments that produced multiple hemoglobin bands (20x HbC/A2, 28x HbS, $11\times$ HbF, and 7x HbA0). The individual horizontal lines for each hemoglobin group represents the mean for the data set (HbC/A2= $6.3 \pm 0.9mm$, HbS= $9 \pm 1mm$, HbF= $10.9 \pm 0.7mm$, and HbA0= $13 \pm 1mm$). The horizontal lines between hemoglobin groups represent statistically significant differences based on one way Analysis of Variance (ANOVA) test (p<0.001). Receiver-operating curves (ROC) were utilized to assess differentiation of different hemoglobin phenotypes based on their traveling distances in the HemeChip (Fig. 5B). Comparison of HbC/A2 to HbS yielded *sensitivity* - 0.90 and *specificity* - 0.89. Comparison of HbS to HbF yielded *sensitivity* = 0.09 and *specificity* = 0.92. Comparison of HbF to HbA0 yielded *sensitivity* = 1.00 and *specificity* = 0.86.

Using the novel HemeChip, we have been able to successfully identify and quantify hemoglobin types C/A2, S, F, and A0. We have shown that the HemeChip can distinguish between different types of hemoglobin disorders, including, homozygous HbSS (SCD), heterozygous HbSA (SCT), and HbSC (hemoglobin SC disease). Separations between hemoglobin bands were visible to the naked eye. Quantitative analysis showed that the HemeChip hemoglobin percentages were comparable to the HPLC and bench-top electrophoresis results.

The HemeChip offers several advantages over the current methods of diagnosing SCD. The first advantage is the low cost of the HemeChip. For less than $2.00, we can produce a HemeChip with all of its necessary reagents. In comparison, our lab currently pays $19.25 per sample for HPLC analysis at the Core Laboratory of University Hospitals Case Medical Center (Cleveland, OH). Similarly, our lab's standard benchtop electrophoresis setup requires approximately 15 times more material in cellulose acetate paper and reagents than the HemeChip. Including the initial investments for these standard systems dramatically increases the cost difference when compared to the HemeChip.

The second advantage is the portability and ease of use of the HemeChip for POC diagnosis. The HemeChip currently requires a power supply, camera, and computer. This means that the HemeChip can be used anywhere with power outlets and does not require a dedicated lab environment. Sample processing on the HemeChip is simple, and involves preparing the sample, loading the buffer, and turning on the power. Although the samples in this paper were primarily analyzed in ImageJ, we have developed a preliminary web-based image analysis software for use with mobile devices. The prototype is discussed and compared to the manual image processing in Fig. 7. Result analysis will simply involve taking a picture of the HemeChip and uploading it to an automated application. This software will further improve the portability and ease of use of the HemeChip. The overall simplicity of the HemeChip allows any user with basic laboratory skills to cheaply and accurately detect SCD, SCT, and Hemoglobin SC disease in comparison with HPLC and benchtop electrophoresis.

The third advantage of the HemeChip is its short runtime. Within 20 minutes, the HemeChip technology can process, analyze, and display the results to the user (not including staining). This significant reduction in diagnostic time allows higher patient throughput in settings where the number of clinical technicians is limited. Since the HemeChip is designed for individual samples, patients can receive their results as soon as the test is finished. In contrast, standard bench-top electrophoresis are often run in batches which delays the results to the patients. This is crucial in areas that lack a system for contacting patients after they have left the POC.

With the use of cord blood samples we have additionally been able to appreciate the high expression of fetal hemoglobin (HbF) in newborn patients. Fetal hemoglobin expression is 50-95% in healthy babies and declines over the course of 6 months. By adulthood less than 1% of the total hemoglobin is HbF. HbF expression is also increased in individuals RBC disorders such as SCD and beta-thalassemia. This high expression of the hemoglobin masks the classic SCD phenotype in many newborns that become symptomatic after 6 months. The HemeChip detects HbF and it can be distinguished from both HbA and HbS. In order to improve the diagnostic precision of the HemeChip apparatus, we have looked to methods in which HbF expression can be reduced in blood samples.

A new protocol is in development in which citrate phosphate is used to precipitate HbA and HbS from RBCs. Following the elusion, adult hemoglobin rises above the cells containing fetal hemoglobin at the bottom of the tube. The supernatant containing adult hemoglobin will then be used to run HemeChip experiments. With this new protocol, the lysis step of RBCs is bypassed and fetal hemoglobin protein concentrations are reduced. To date, we have found that the exposure of citrate phosphate reduces the HbF protein concentration by 50% in uncentrifuged samples.

Example 2

Microfluidic Biochip Fabrication

PMMA top parts are prepared by cutting an inlet and outlet (0.61 mm in diameter and 26 m apart) using a VersaLASER system (Universal Laser Systems Inc., Scottsdale, AZ). Double sided adhesive (DSA) film (iTapestore, Scotch Plains, NJ) is cut to fit the size of the PMMA part and 28x4 mm channels. DSA is then attached to the PMMA top part to include an inlet and outlet between the outline of the DSA film. Gold Seal glass slide is then assembled with the PMMA-DSA structure to form a microfluidic channel.

Surface Chemistry

GMBS stock solution is prepared by solving 25 mg of GMBS in 0.25 mL DMSO, and stock solution is diluted with ethanol to obtain 0.28% v/v GMBS working solution. FN is diluted with PBS to create a (1:10) FN working solution. BSA solution is prepared by solving 3 mg of lyophilized BSA in 1 mL PBS.
The channels are washed with 30 $\mu$L of PBS and ethanol after assembly. Next, 20 $\mu$L of cross-linker agent GMBS working solution is injected into the channels twice and incubated for 15 min. at room temperature. Following GMBS incubation, channels are washed twice with 30 $\mu$L of ethanol and PBS. Next, 20 $\mu$L of FN solution is injected into the channels and incubated for 1.5 h at room temperature.
The surface is then passivated by injecting 30 $\mu$L of BSA solution and overnight incubation at 4°C. Before processing blood samples, channels are rinsed with PBS.

Blood Processing

Discarded de-identified patient blood samples were obtained from University Hospital's Hematology and Oncology Division under institutional review board (1RB) approval. Blood samples were collected into EDTA-containing purple cap Vacutainer tubes. Before using in experiments, blood samples were aliquoted into sealed microtubes and sealed syringes to minimize exposure to ambient air. Blood flow through the channels and following FCSB flow steps are applied using New Era NE-300 syringe pump system (Farmingdale, NY). Blood samples are kept sealed and upright in 1 mL disposable syringes before and during injection into microchannels. Next, blood is introduced into microchannels at 28.5 $\mu$L/min until the channel is filled with blood and then 15 $\mu$L of blood sample is injected at a flow rate of 2.85 $\mu$L/min. Next, the syringe is changed and 120 $\mu$L of FCSB at a flow rate of 10 $\mu$L/min is introduced into the channel to remove the non-adhered cells. Adhered RBCs in channels are visualized using an inverted fluorescent microscope (Olympus IX83) and fluorescent microscopy camera (EXi Blue EX1-BLU-R-F-M-14-C). During real time microscope imaging and high resolution video recording at 7 fps rate, controlled fluid flow with stepwise increments are applied until RBC detachment is observed from the microchannel surface.

Microfluidic Channel Visualization and Image Processing

An Olympus 1X83 inverted motorized microscope with Olympus Cell Sense live-cell imaging and analysis software is used to obtain real-time microscopic recordings in this study. Olympus (20x/0.45 ph2 and 40x/0.75 ph3) long working distance objective lenses are utilized for phase contrast imaging of single RBCs in microchannels. (Fig. 9A). Videos are recorded at 7fps and converted to single frame images for further processing and analysis. Cell dimensions are analyzed by using Adobe Photoshop software (San Jose, CA).
Adhered RBCs were analyzed in terms of biophysical properties in flow in the recorded images, and we utilize the free flowing cells (appearing as a blurry line at 1.5 ms camera exposure time) for determining local flow velocities. (Fig. 10A). In addition, cellular adhesion, cellular deformation in flow, and cellular detachment re analyzed in the recorded sequential images taken at 7 frames per second.

Data Analysis

Flow velocity on the adhered RBCs are calculated using Equations 3 and 4 describing pressure-driven flow in a rectangular channel:

$$u_x(y,z) = \frac{16h^2}{\eta\pi^3}\left(-\frac{dp}{dx}\right)\sum_{n=1,3,5\ldots}^{\infty}(-1)^{(n-1)/2}\left[1-\frac{\cosh\left(\frac{n\pi z}{2h}\right)}{\cosh\left(\frac{n\pi w}{2h}\right)}\right]\frac{\cos\left(\frac{n\pi y}{2h}\right)}{n^3} \quad (3)$$

$$Q = \frac{4}{3\eta}wh^3\left(-\frac{dp}{dx}\right)\left[1-\frac{192}{\pi^5}\frac{h}{w}\sum_{n=1,3,5\ldots}^{\infty}\frac{1}{n^5}\tanh\left(\frac{n\pi w}{2h}\right)\right], \quad (4)$$

where $x$, $y$ and $z$ are the principal axes, $h$ and $w$ are the channel height and width, $n$ is the fluid viscosity $dp/dx$ is the pressure change along the $x$ axis, and $Q$ is the volumetric flow rate. See, Table 1.

Table 1 Parameters used in drag force quantification at RBC detachment

|  | Value |
| --- | --- |
| Measured RBC Width | 4.47-8 ($\mu$M) |
| HBA | 6.73-8 ($\mu$M) |
| HbS deformable | 4.8-7.84 ($\mu$M) |
| HbS non-deformable | 4.47-7.36 ($\mu$M) |
| RBC Thickness | 2.25 ($\mu$M) |
| Microchannel Heights | 50 ($\mu$M) |
| Microchannel Width | 4 (mm) |
| Buffer Density | 993 Kg/m$^3$ |
| Buffer Viscosity | 0.001 Pa-d |

Drag force applied on the adhered RBCs is calculated using drag force equation (Equation 5):

$$F_d = \left(\frac{1}{2}\right)\rho u_x^2 C_d A \quad (5)$$

where $F_d$ is the drag force, $p$ is the fluid density, $C_d$ is the drag coefficient, and $A$ is the reference area. $C_d$ is calculated as $13.6/Re$, where $Re$ is the Reynolds number, according to the circular disk parallel to flow assumption of cell at low Reynolds number flow. Munson et al., In: Fundamentals of Fluid Mechanics. 7 ed.; John Wiley & Sons Canada: Mississauga, ON, Canada, 2012; p 792. $A$, reference area is calculated by using the typical RBC thickness and the measured RBC width at detachment. Shear stress ($T$) on the adhesion surface is calculated using Equation 6:

$$T = 6nQ/wh^2 \quad (6)$$

Statistical Analysis

Data obtained in this study are reported as mean $\pm$ standard error of the mean. Cell aspect ratio, aspect ratio change (deformability), flow rate, shear stress, and drag forces are statistically assessed (Minitab 16 software, Minitab Inc., State College, PA) using Analysis of Variance (ANOVA) with Fisher's post hoc test for multiple comparisons (n=3-6 blood samples per group). Statistical significance is set at 95% confidence level for all tests (p< 0.05). Error bars in figures represent the standard error of the mean.

Data Clustering: The relationship between the individual components of the complete blood count and the number of adhered RBCs was analyzed using K-means clustering method. The patients with HbSS were clustered into two groups using K-means and the resultant groups were evaluated for differences in disease severity. Single components of complete blood count as well as multiple components were used in K-means clustering to identify the two sub-groups. Once the sub-groups were identified, the difference between the numbers of adhered RBCs between these groups were tested for statistical significance using one way ANOVA test. The testing level (alpha) was set as 0.05 (two-sided). The component or components that lead to the significant sub-groups in terms of the differences between the numbers of adhered RBCs were reported in this paper. The K-means clustering was performed using Matlab® (The MathWorks, Inc, Natick, MA).

Receiver-Operating Characteristic (ROC) Curves: Receiver-operating curves were used to determine the SCD-Biochip's accuracy of differentiation between hemoglobin phenotypes. The curves were generated using Matlab® (The MathWorks, Inc, Natick, MA). In addition to the area under the curve, sensitivity, specificity, positive and negative likelihood ratios, and positive and negative predictive values were calculated as follows: Sensitivity was calculated as # true positives / (# true positives + # false negatives), specificity as # true negatives / (# true negatives + # false positives). Positive likelihood ratio was defined as sensitivity / (1-specificity). Negative likelihood ratio was (1-sensitivity) / specificity. Positive predictive value was # true positives / (# true positives + # false positives), and negative predictive value was # true negatives / (# true negatives + # false negatives).

Example 3

This example describes a microfluidic biochip that includes at least one etched microchannel comprising a fibronectin (FN) functionalized glass surface. The biochip further includes a poly(methyl methacrylate) plastic top having etched inlet ports and etched outlet ports. The top further comprises a double sided adhesive film in the middle that defines the outlines and the width of the microchannel (e.g., approximately 50 $\mu$m). (Figs. 9A & 13C). Further, the microfluidic system may be placed on an Olympus IX83 inverted motorized microscope stage for high resolution live single cell image recording and analysis.

FN circulates in plasma and is present in the endothelial cell membrane. FN is believed to be an adhesive glycoprotein that has been shown to play a role in HbS RBC adhesion to the endothelial wall. Kasschau et al., Blood 87(2):771-780 (1996); Wick et al., Current opinion in hematology 3(2): 118-124 (1996); Mosher, D. F., Annual Review of Medicine 35:561-575 (1984); and Kumar et al., Blood 88(11):4348- 4358 (1996). FN was immobilized on microfluidic channel surfaces to mimic, in part, endothelial wall characteristics. The microfluidic design allows precise control of flow velocities similar to physiological conditions in microvasculature, resulting in laminar flow conditions with straight and parallel streamlines on the surface. Lipowsky, H. H., Microcirculation 12(1):5-15 (2005). A laminar flow profile in microfluidic channels enables interaction of flowing RBCs with FN immobilized surface and enables attachment of those RBCs with increased adhesive properties. (Fig. 9B). The resulting data show an adhesion of a morphologically heterogeneous RBC population in blood samples from subjects with HbS. (Fig. 9C). This adhesion was not observed when testing HbA blood samples (data not shown). Adhered RBCs included mildly sickled (Fig. 9C(i)), moderately sickled (Fig. 9C(ii) and highly sickled (Fig. 9C(iii)) cell morphologies within the same field from a single HbS-containing blood sample.

These data were analyzed for aspect ratio (AR) and deformability of single HbA- or HbS-containing RBCs in three conditions: (1) no flow, (2) flow, and (3) at detachment. The data demonstrated two sub-groups of HbS-containing RBCs in terms of deformability: HbS deformable and HbS non-deformable. (Fig. 10A). Cell aspect ratio change, with respect to no flow condition, is used as a measure of deformability, in which a greater change in cell aspect ratio translates to more deformability and hence, less stiffness. Flow velocities are increased in a step-wise manner. Cell deformability for each RBC is assessed at a maximal flow velocity just prior to detachment in a time-lapse experiment. This analysis provided a maximum deformation estimate of an adhered cell. (Fig. 10B & 10C).

HbA-containing RBCs show significantly greater cell aspect ratios (*i.e.,* for example, circularity) than do HbS-containing RBCs at no-flow (p<0.05, one way ANOVA with Fisher's post-hoc test). The aspect ratio of HbA RBCs significantly decreases in the presence of flow (p<0.05, one way ANOVA with Fisher's post-hoc test), implying higher deformability. HbS-containing deformable RBCs present a significant decrease in aspect ratio only at the detachment instant, compared to no-flow conditions (p<0.05, one way ANOVA with Fisher's post-hoc test). Indeed, the aspect ratio of HbS non-deformable RBCs does not change in any flow condition (p>0.05, one way ANOVA with Fisher's post-hoc test. (Fig. 10B).

The HbS deformable RBCs display a significantly greater cell aspect ratio at no-flow and significantly greater deformability at detachment as compared with HbS non-deformable RBCs (p<0.05, one way ANOVA with Fisher's post-hoc test). (Figs. 10B & 10C). The deformability of HbA-containing RBCs is significantly greater than HbS-containing RBCs in all flow conditions (p<0.05, one way ANOVA with Fisher's post-hoc test). The deformability of both HbA and HbS-containing deformable RBCs is significantly different when measured during flow and when measured at detachment (p<0.05, one way ANOVA with Fisher's post-hoc test). However, under these same conditions, HbS nondeformable RBCs do not display any significant difference in deformability during this interval (p>0.05, one way ANOVA with Fisher's post-hoc test). While adhered on a surface that mimics features of the normal blood stream in SCD (*i.e.,* for example, an FN-functionalized surface), HbS-containing RBCs are heterogeneous in aspect ratio and in deformability. (Fig. 10C).

In another example, microfluidic channels were composed of a glass surface functionalized with FN or LN, a Poly(methyl methacrylate) plastic top (encompassing inlets and outlets), and sandwiched 50 $\mu$m thick double sided adhesive tape that defines the height and shape of the microchannels (Fig. 13). FN is a glycoprotein that circulates in plasma and is present in the endothelial cell membrane. FN plays a role in SCD RBC adhesion, via RBC integrin $\alpha 4\beta 1$ interaction with the endothelial wall. LN is sub-endothelial and binds to an important RBC surface protein from the immunoglobulin superfamily, BCAM/LU, which is phosphorylated during beta-adrenergic stimulation.

The number of adhered RBCs was quantified inside the FN or LN immobilized microfluidic channels. We observed abnormal adhesion of RBCs in blood samples from subjects with SCD. On the other hand, adhesion of RBCs in blood samples from normal subjects was negligible (not shown). High resolution phase-contrast images of FN and LN coated microchannel surface inside the SCD-Biochip revealed heterogeneous sickle morphologies of adhered RBCs. A range of RBC adhesion was observed in patients with various clinical phenotypes.

We analyzed the number of adhered RBCs per unit area (32 mm2) in FN and LN functionalized microchannels in blood samples from subjects with HbAA, compound heterozygous HbSC or HbS$\beta$+-thalassemia (HbSC/S$\beta$+), or homozygous HbSS, using high resolution images from microfluidic channels (Fig. 14). Among blood samples with different hemoglobin phenotypes, the number of adhered RBCs was significantly higher in HbSS > HbSC/S$\beta$+ > HbAA blood samples in FN (P=0.023 and P=0.002, respectively) and LN (P=0.024 and P=0.011, respectively, Figs. 14C & 14D). Furthermore, HbSC/S$\beta$+ blood samples displayed a significantly higher number of adhered RBCs than HbAA blood samples in both FN (P=0.002) and LN (P=0.027) functionalized microchannels (Figs. 14C & 14D).

Next we plotted receiver operating-characteristic (ROC) curves to assess the SCD-Biochip's ability to accurately determine hemoglobin phenotypes through adhesion (Figs. 14E & 14F). For selected thresholds of adhered RBC numbers (Figs. 10E & 10F), we observed 0.93 true-positive rate and 0.00 false-positive rate for differentiating between HbSS and HbAA phenotypes. Area under the curve for differentiating between HbSS and HbAA was more than 0.85 both for FN and LN. These results demonstrate the ability of the SCD Biochip to discriminate amongst hemoglobin phenotypes through cellular adhesion. These data may further support the role that abnormal cellular adhesion plays in accounting for SCD phenotypes, in which HbSS is more hemolytic and HbSC less so.

Because of the ameliorative role of HbF in SCD, we investigated RBC adhesion in HbSS blood samples with low (<%8) and high (>%8) HbF levels (Fig. 15). When compared, number of adhered RBCs was significantly higher in blood samples from subjects with low HbF than high HbF levels in both FN (P=0.015, Fig. 15A) and LN (P=0.022, Fig. 15B) functionalized microchannels. These findings establish the base ground for the SCD-Biochip as an *in vitro* adhesion assay for functional phenotypes of SCD, and the impact on these phenotypes of novel therapies.

We utilized K-means clustering analysis to identify sub-groups among HbSS patients based on single components of the standard of care blood test results and hemoglobin testing results. We analyzed RBC adhesion to FN and LN microchannels in HbSS blood samples from patients with various clinical phenotypes, including high and low lactate dehydrogenase (LDH), platelet counts (plts), and reticulocyte counts (retics) (Fig. 16). We observed significantly higher number of adhered RBCs to both FN (P=0.0003) and LN (P=0.003) immobilized microchannels in blood samples from patients with high LDH levels (>500 u/L) compared to patients with low LDH levels (<500 u/l) (Figs. 16A & 16C). Also, RBCs in blood samples from patients with high plts (>320 109/L) showed significantly higher adhesion to FN immobilized microchannels (P=0.046) than patients with low plts (<320 109/L) (Fig. 16B). Furthermore, we observed significantly higher RBC adhesion to LN functionalized microchannels (P=0.014) in blood samples from patients with high retics (>320 109/L) compared to patients with low retics (<320 109/L) (Fig. 16D).

We, then, analyzed the heterogeneity of adhered RBCs in FN functionalized microchannels (Fig. 17). Morphology and number of adhered RBCs in HbSS blood samples were quantified after controlled detachment of cells at stepwise increased flow velocities of 0.8 mm/s, 3.3 mm/s, and 41.7 mm/s (Figs. 17A-C). Based on morphological characterization, adhered RBCs were categorized as deformable (Fig. 17D) and non-deformable (Fig. 13E) RBCs. Percentages of deformable and non-deformable RBCs of total adhered RBCs at 0.8 mm/s flow velocity were calculated (Fig. 17F). Deformable and non-deformable RBC percentages were not significantly different at 0.8 mm/s (P=0.266). On the other hand, percentage of non-deformable RBCs were significantly higher than the percentage of non-deformable RBCs at 41.7 mm/s (P=0.047, Fig. 17F). Moreover, the percentage of deformable RBCs were significantly lower at 3.3 mm/s and 41.7 mm/s compared to 0.8 mm/s (P=0.001 and P<0.001, respectively). Also, the percentage of deformable RBCs were significantly lower at 41.7 mm/s than 3.3 mm/s (P<0.001). However, the only significant difference in the percentage of non-deformable RBCs were observed between 0.8 mm/s and 41.7 mm/s (P=0.003, Fig. 17F). Furthermore, we observed a significant association between adhered non-deformable RBCs (%) and serum LDH levels in our subjects (Fig. 13G, Pearson correlation coefficient of 0.79, p<0.005). These results indicate a morphological and qualitative heterogeneity in adhered RBCs and an association between hemolysis and adherent non-deformable RBCs.

From the above description of the invention, those skilled in the art will perceive improvements, changes and modifications. Such improvements, changes, and modifications are within the skill of the art and are intended to be covered by the appended claims. All patents and publications identified herein are incorporated by reference in their entirety.

REFERENCES

1. Sickle-Cell Disease in the African Region: Current Situation and the Way Forward. AFR/RC56/17, 2006, World Health Organization Regional Committee for Africa, WHO; Addis Adaba, Ethiopia.
2. Sickle-cell disease: a strategy for the WHO African Region: Report of the Regional Director. AFR/RC60/8, 2010, World Health Organization Regional Office for Africa, WHO; Geneva, Switzerland.
3. Sickle Cell Disease and other Hemoglobin Disorders Fact Sheet, 2011, World Health Organization.
4. Grosse, S.D., I. Odame, H.K. Atrash, D.D. Amendah, F.B. Piel, and T.N. Williams, Sickle cell disease in Africa: a neglected cause of early childhood mortality. Am J Prev Med, 2011. 41(6 Suppl 4): p. S398-405.
5. Odame, I., Perspective: We need a global solution. Nature, 2014. 515(7526): p. S10-S10.
6. Makani, J., S.E. Cox, D. Soka, A.N. Komba, J. Oruo, H. Mwamtemi, P. Magesa, S. Rwezaula, E. Meda, J. Mgaya, B. Lowe, D. Muturi, D.J. Roberts, T.N. Williams, K. Pallangyo, J. Kitundu, G. Fegan, F.J. Kirkham, K. Marsh, and C.R. Newton, Mortality in sickle cell anemia in Africa: a prospective cohort study in Tanzania. PLoS One, 2011. 6(2): p. e14699.
7. Chiodini, P.L., K. Bowers, P. Jorgensen, J.W. Barnwell, K.K. Grady, J. Luchavez, A.H. Moody, A. Cenizal, and D. Bell, The heat stability of Plasmodium lactate dehydrogenase-based and histidine-rich protein 2-based malaria rapid diagnostic tests. Trans R Soc Trop Med Hyg, 2007. 101(4): p. 331-7.

Before we go on to set out the claims, we first set out the following clauses describing some prominent features of certain embodiments of the invention:

Clause 1: An electrophoresis biochip for detecting hemoglobin disorders, comprising:

a housing that includes a microchannel that extends from a first end to a second end of the housing, the microchannel containing cellulose acetate paper that is at least partially saturated with an alkaline buffer solution;
a first buffer port and a second buffer that extend, respectively, through the first end and second of the housing to the microchannel and cellulose acetate paper, the first buffer port and the second buffer port being capable of receiving the alkaline buffer solution that at least partially saturates the cellulose acetate paper;
a sample loading port for receiving a blood sample, the sample loading port extending through the first end of the housing to the microchannel and cellulose acetate paper; and
a first electrode and a second electrode for generating an electric field across the cellulose acetate paper, the first electrode and second electrode extending, respectively, through the first buffer port and the second port to the cellulose acetate paper.

Clause 2: The electrophoresis biochip of clause 1, further comprising an imaging system for visualizing and quantifying hemoglobin variant migration along the cellulose acetate paper for blood samples introduced into the sample loading port.

Clause 3: The electrophoresis biochip of clause 1, the housing including a viewing area for visualizing the cellulose acetate paper and hemoglobin variant migration.

Clause 4: The electrophoresis biochip of clause 1, wherein the first electrode and the second electrode are connected to a power supply, the power supply generating an electric field of about 1V to about 400V.

Clause 5: The electrophoresis biochip of clause 4, wherein the voltage applied to the biochip by the electrodes does not exceed 250V.

Clause 6: The electrophoresis biochip of clause 1, wherein the blood sample introduced into the sample loading port is less than 10 microliters.

Clause 7: The electrophoresis biochip of clause 1, wherein the buffer solution comprises alkaline tris/Borate/EDTA buffer solution.

Clause 8: The electrophoresis biochip of clause 1, wherein the first electrode and the second electrode comprise graphite electrodes.

Clause 9. The electrophoresis biochip of clause 1, wherein the housing comprises a top cap, a bottom cap, and a channel spacer interposed between the top cap and the bottom cap, the channel spacer defining the channel in the housing.

Clause 10: The electrophoresis biochip of clause 1, wherein the top cap, bottom cap, and channel spacer comprise at least one of glass or plastic.

Clause 11: The electrophoresis biochip of clause 2, the imaging system comprising a mobile phone imaging system to visualize and quantify hemoglobin variant migration.

Clause 12: The electrophoresis biochip of clause 11, wherein the mobile phone imaging system includes a mobile telephone that is used to image hemoglobin variant migration and a software application that recognizes and quantifies the hemoglobin band types and thicknesses to make a diagnostic decision.

Clause 13: The electrophoresis biochip of clause 12, wherein the hemoglobin band types comprise hemoglobin types C/A, S, F, and A0.

Clause 14: The electrophoresis biochip of clause 11, diagnosing whether the subject has hemoglobin genotypes HbSS, HbSA, HbSC, or HbA2.

Clause 15: The electrophoresis biochip of clause 1, diagnosing whether the subject has sickle cell disease.

Clause 16: An electrophoresis biochip system for diagnosing sickle cell disease in a subject, comprising:

a housing that includes a microchannel that extends from a first end to a second end of the housing, the microchannel containing cellulose acetate paper that is at least partially saturated with an alkaline buffer solution;
a first buffer port and a second buffer that extend, respectively, through the first end and second of the housing to the microchannel and cellulose acetate paper, the first buffer port and the second buffer port being capable of receiving the alkaline buffer solution that at least partially saturates the cellulose acetate paper;
a sample loading port for receiving a blood sample, the sample loading port extending through the first end of the housing to the microchannel and cellulose acetate paper;
a first electrode and a second electrode for generating an electric field across the cellulose acetate paper, the first electrode and second electrode extending, respectively, through the first buffer port and the second port to the cellulose acetate paper; and
an imaging system for visualizing and quantifying hemoglobin variant migration along the cellulose acetate paper for blood samples introduced into the sample loading port and determining whether the subject has sickle cell disease.

Clause 17: The electrophoresis biochip system of clause 16, the housing including a viewing area for visualizing the cellulose acetate paper and hemoglobin variant migration.

Clause 18: The electrophoresis biochip system of clause 16, wherein the first electrode and the second electrode are connected to a power supply, the power supply generating an electric field of about 1V to about 400V.

Clause 19: The electrophoresis biochip system of clause 18, wherein the voltage applied to the biochip by the electrodes does not exceed 250V.

Clause 20: The electrophoresis biochip system of clause 16, wherein the blood sample introduced into the sample loading port is less than 10 microliters.

Clause 21: The electrophoresis biochip system of clause 16, wherein the buffer solution comprises alkaline tris/Borate/EDTA buffer solution.

Clause 22: The electrophoresis biochip system of clause 16, wherein the first electrode and the second electrode comprise graphite electrodes.

Clause 23: The electrophoresis biochip system of clause 16, wherein the housing comprises a top cap, a bottom cap, and a channel spacer interposed between the top cap and the bottom cap, the channel spacer defining the channel in the housing.

Clause 24: The electrophoresis biochip system of clause 16, wherein the top cap, bottom cap, and channel spacer comprise at least one of glass or plastic.

Clause 25: The electrophoresis biochip of clause 16, imaging system comprising a mobile phone imaging system to visualize and quantify hemoglobin variant migration.

Clause 26: The electrophoresis biochip of clause 16, wherein the mobile phone imaging system includes a mobile telephone that is used to image hemoglobin variant migration and a software application that recognizes and quantifies the hemoglobin band types and thicknesses to make a diagnostic decision.

Clause 27: The electrophoresis biochip of clause 16, wherein the hemoglobin band types comprise hemoglobin types C/A, S, F, and A0.

Clause 28: A method comprising:
providing an electrophoresis biochip system that includes:

a housing that includes a microchannel that extends from a first end to a second end of the housing, the microchannel containing cellulose acetate paper that is at least partially saturated with an alkaline buffer solution;
a first buffer port and a second buffer that extend, respectively, through the first end and second of the housing to the microchannel and cellulose acetate paper, the first buffer port and the second buffer port being capable of receiving the alkaline buffer solution that at least partially saturates the cellulose acetate paper;
a sample loading port for receiving a blood sample, the sample loading port extending through the first end of the housing to the microchannel and cellulose acetate paper;
a first electrode and a second electrode for generating an electric field across the cellulose acetate paper, the first electrode and second electrode extending, respectively, through the first buffer port and the second port to the cellulose acetate paper; and
an imaging system for visualizing and quantifying hemoglobin variant migration along the cellulose acetate paper for blood samples introduced into the sample loading port.
introducing a blood sample from a subject into the sample loading port, the blood sample comprising at least one blood cell; and
imaging hemoglobin bands formed on the cellulose acetate paper with the imaging system to determine hemoglobin phenotype for said subject.

Clause 29: The method of clause 28, wherein the hemoglobin phenotype is selected from the group consisting of HbAA, HbSA, HbSS, HbSC, and HbA2.

Clause 30: The method of clause 29, wherein said HbAA hemoglobin phenotype diagnoses the subject as normal.

Clause 31: The method of clause 29, wherein said HbSA hemoglobin phenotype diagnoses the subject as having a sickle cell trait.

Clause 32: The method of clause 29, wherein said HbSS hemoglobin phenotype diagnoses the subject as having a sickle cell disease.

Clause 33: The method of clause 29, wherein said HbSC hemoglobin phenotype diagnoses the subject as having a hemoglobin SC disease.

Clause 34: The method of clause 29, wherein said HbA2 hemoglobin phenotype diagnoses the subject as having thalassemia.

Clause 35: The method of clause of 28, the housing including a viewing area for visualizing the cellulose acetate paper and hemoglobin variant migration.

Clause 36: The method of clause 28, wherein the first electrode and the second electrode are connected to a power supply, the power supply generating an electric field of about 1V to about 400V.

Clause 37: The method of clause 36, wherein the voltage applied to the biochip by the electrodes does not exceed 250V.

Clause 38: The method of clause 28, wherein the blood sample introduced into the sample loading port is less than 10 microliters.

Clause 39: The method of clause 28, wherein the buffer solution comprises alkaline tris/Borate/EDTA buffer solution.

Clause 40: The method of clause 28, wherein the first electrode and the second electrode comprise graphite electrodes.

Clause 41: The method of clause 28, the imaging system comprising mobile phone imaging system to visualize and quantify hemoglobin variant migration.

Clause 42: The method of clause 41, the mobile phone imaging system includes a mobile telephone that is used to image hemoglobin variant migration and a software application that recognizes and quantifies the hemoglobin band types and thicknesses to make a diagnostic decision.

Clause 43: The method of clause 28, wherein the hemoglobin band types comprise hemoglobin types C/A, S, F, and A0.

Clause 44: A microfluidic biochip device, comprising:
a housing that includes at least one microchannel that defines at least one cell adhesion region, the at least one cell adhesion region being coated with at least one bioaffinity ligand that adheres a cell of interest when a fluid containing cells is passed through the at least one microchannel and an imaging system that measures the quantity of cells adhered to the at least one bioaffinity ligand within the at least one microchannel when the fluid is passed through the channels, the bioaffinity ligands comprising at least one of fibronectin, laminin, selectin, von Willebrands' Factor, thrombomodulin or a C146 antibody.

Clause 45: The device of clause 44, wherein the imaging system comprises a lensless imaging system.

Clause 46: The device of clause 45, wherein the lensless imaging system comprising a charged coupled device sensor and a light emitting diode.

Clause 47: The device of clause 44, wherein the cells are blood cells obtained from the subject.

Clause 48: The device of clause 44, wherein the imaging system quantifies the adhered cells in each respective channel to monitor heath of a subject from which the cells are obtained.

Clause 49: The device of clause 44, wherein the imaging system quantifies the adhered cells in each respective channel to monitor the progression of a disease of a subject from which the cells are obtained.

Clause 50: The device of clause 49, wherein the disease is sickle cell disease.

Clause 51: The device of clause 44, wherein the imaging system quantifies the adhered cells in each channel to measure the efficacy of a therapeutic treatment administered to a subject from which the cells are obtained.

Clause 52: The device of clause 44, the housing including a plurality of microchannels, each microchannel a separate cell adhesion region coated with at least one bioaffinity ligand.

Clause 53: The device of clause 52, at least two microchannels including different bioaffinity ligands.

Clause 54: The device of clause 52, at least three microchannels including different bioaffinity ligands.

Clause 55: The device of clause 52, the plurality of the microchannels including the same bioaffinity ligands.

Clause 56: The device of clause 52, at least one microchannel including at least two different bioaffinity ligands coated on the cell adhesion region of the microchannel.

Clause 57: The device of clause 52, wherein different bioffinity ligands are located at different positions within the cell adhesion region of at least one microchannel.

Clause 58: The device of clause 44, wherein the housing is a multilayer structure formed of a base layer, an intermediate layer, and a cover layer, the at least one microchannel being formed in the intermediate layer.

Clause 59: The device of clause 44, the housing including an inlet port and an outlet port in fluid communication respectively with a first end and a second end of each microchannel.

Clause 60: The device of clause 44, wherein the at least one microchannel is sized to accept microliter or milliliter volumes of blood or a solution containing cells to be adhered.

Clause 61: The device of clause 44, wherein at least one of the laminin, the selectin, the von Willebrands' Factor, the thrombomodulin and the C146 antibody are localized at different positions along the at least one microchannel.

Clause 62: A method, comprising:

providing a microfluidic biochip device, comprising a housing that includes at least one microchannel that defines at least one cell adhesion region, the at least one cell adhesion region being coated with at least one bioaffinity ligand that adheres a cell of interest when a fluid containing cells is passed through the at least one microchannel and an imaging system that measures the quantity of cells adhered to the at least one bioaffinity ligand within the at least one microchannel when the fluid is passed through the channels, the bioaffinity ligands comprising at least one of fibronectin, laminin, selectin, von Willebrands' Factor, thrombomodulin or a C146 antibody; introducing a blood sample derived from a subject into the microchannel, wherein the blood sample comprises at least one blood cell; and imaging the quantity of cells adhered to the at least one bioaffinity ligand within the at least one microchannel.

Clause 63: The method of clause 62, wherein the imaging system comprises a lensless imaging system.

Clause 64: The method of clause 63, wherein the lensless imaging system comprising a charged coupled device sensor and a light emitting diode.

Clause 65: The method of clause 62, wherein the quantity of adhered cells in each respective channel is indicative of the heath of the subject from which the cells are obtained.

Clause 66: The method of clause 62, wherein the quantity of adhered cells in each respective channel is indicative of the progression of a disease of the subject from which the cells are obtained.

Clause 67: The method of clause 66, wherein the disease is sickle cell disease.

Clause 68: The method of clause 62, wherein the quantity of adhered cells in each respective channel is indicative of the efficacy of a therapeutic treatment administered to a subject from which the cells are obtained.

**Claims**

1. An electrophoresis system comprising:

a housing that includes a microchannel that extends from a first end to a second end of the housing, the microchannel containing an electrophoresis sieving medium that includes cellulose acetate paper at least partially saturated with a buffer solution;
a sample loading port introducing a sample to the microchannel and sieving medium; and
a first electrode and a second electrode for generating an electric field along a length of the sieving medium, wherein the generated electric field induces migration and separation of components of the sample introduced to the electrophoresis sieving medium into one or more bands on the sieving medium,

an imaging system structured to detect the one or more bands of the components of the sample on the electrophoresis strip caused by the generated electric field and to generate band detection data based on the one or more bands of the components of the sample; and

an imaging processor programmed to receive the band detection data to determine one or more band characteristics corresponding to the one or more bands of the components of the sample and identify components in the sample.

2. The electrophoresis system of claim 1, wherein the imaging system is portable and computer interfaceable and is used for visualizing and quantifying the one or more band migration along the sieving medium.

3. The electrophoresis system of claim 2, wherein the portable, computer interfaceable imaging system includes a camera that is used to image one or more band migration and the imaging system includes a software application that recognizes separation of components of the sample.

4. The electrophoresis system of claim 3, the housing including an optically clear area for visualizing the sieving medium and migration and separation of components of the sample.

5. The electrophoresis system of claim 4, wherein the optically clear area includes optically transparent glass or plastic.

6. The electrophoresis system of claim 5, wherein the sample is a blood sample and the first electrode and second electrode generate an electric field effective to promote migration and separation of hemoglobin variants in the blood sample.

7. The electrophoresis system of claim 6, wherein the camera is used to image one or more bands corresponding to the hemoglobin variant migration and the software recognizes and quantifies the band types and/or density to aid screening decision or diagnosis.

8. The electrophoresis system of claim 1, wherein the voltage applied by the first electrode and the second electrode is in the range of 1V to 400 V.

9. The electrophoresis system of claim 1, wherein the sample introduced to the sieving medium is less than 10 microliters.

10. The electrophoresis system of claim 1, wherein the housing comprises a top cap, a bottom cap, and a cavity interposed between the top cap and the bottom cap, the cavity defining the microchannel in the housing.

Figs. 1A-E

# A

i

ii

iii

# B

i

**Blood Sample**

**Cellulose
Acetate**

**Buffer**

ii

**Lead
Electrodes**

iii

**HbS HbA**

Figs. 2A-B

Figs. 3A-B

Figs. 4A-F

Figs. 5A-B

Fig. 6A

Fig. 6B

Fig. 6C

Fig. 7

A

Fig. 8A

B

Fig. 8B

Fig. 9A

Fig. 9B

Fig. 9C

Fig. 10A

Figs. 10B-C

Fig. 11A

Fig. 11B

Fig. 11C

Figs. 11D-E

Figs. 12A-L

Figs. 13A-D

Figs. 14A-F

Figs. 15A-B

Figs. 16A-D

Figs. 17A-G

Europäisches Patentamt

European Patent Office

Office européen des brevets

# EUROPEAN SEARCH REPORT

Application Number

EP 22 15 6878

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2010/181199 A1 (SUGIYAMA KOJI [JP] ET AL) 22 July 2010 (2010-07-22) * paragraphs [0003], [0035], [0037], [0055], [0065], [0077] – [0091] * | 1-10 | INV. C12M1/42 G01N33/483 G01N27/447 |
| A | US 2004/256230 A1 (YAGER PAUL [US] ET AL) 23 December 2004 (2004-12-23) * paragraphs [0041], [0042], [0044], [0105], [0115] – [0117], [0166]; figures 1,3a,3b * | 1-10 | |
| A | WO 96/33410 A1 (BIOMETRIC IMAGING INC [US]) 24 October 1996 (1996-10-24) * page 17, line 29 – page 19, line 4 * | 1-10 | |
| A | TANG ET AL: "Hemoglobin electrophoresis", CLINICAL IMMUNOLOGY NEWSLETTER, ELSEVIER, US, vol. 13, no. 8, 1 August 1993 (1993-08-01) , pages 93-97, XP023988033, ISSN: 0197-1859, DOI: 10.1016/0197-1859(93)90016-D [retrieved on 1993-08-01] * the whole document * | 1-10 | TECHNICAL FIELDS SEARCHED (IPC) G01N |

The present search report has been drawn up for all claims

| Place of search | Date of completion of the search | Examiner |
|---|---|---|
| Munich | 3 August 2022 | Schneiderbauer, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 15 6878

This annex lists the patent family members relating to the patent documents cited in the above-mentioned European search report.
The members are as contained in the European Patent Office EDP file on
The European Patent Office is in no way liable for these particulars which are merely given for the purpose of information.

03-08-2022

| Patent document cited in search report | | Publication date | Patent family member(s) | | Publication date |
|---|---|---|---|---|---|
| US 2010181199 | A1 | 22-07-2010 | CN | 102077085 A | 25-05-2011 |
| | | | EP | 2315015 A1 | 27-04-2011 |
| | | | JP | 2014145775 A | 14-08-2014 |
| | | | JP | WO2010010858 A1 | 05-01-2012 |
| | | | US | 2010181199 A1 | 22-07-2010 |
| | | | WO | 2010010858 A1 | 28-01-2010 |
| US 2004256230 | A1 | 23-12-2004 | EP | 1190241 A2 | 27-03-2002 |
| | | | JP | 2003501639 A | 14-01-2003 |
| | | | US | 2004256230 A1 | 23-12-2004 |
| | | | US | 2012138469 A1 | 07-06-2012 |
| | | | WO | 0074850 A2 | 14-12-2000 |
| WO 9633410 | A1 | 24-10-1996 | AU | 5553496 A | 07-11-1996 |
| | | | US | 5843680 A | 01-12-1998 |
| | | | WO | 9633410 A1 | 24-10-1996 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62030964

**Non-patent literature cited in the description**

- U.S. Preventive Services Task Force. Screening for Hemoglobinopathies. Guide to Clinical Preventive Services. 1996, 485-494
- **MODELL et al.** Global epidemiology of haemoglobin disorders and derived service indicators. *Bulletin of the World Health Organization,* 2008, vol. 86, 480-487
- **MAKANI et al.** Mortality in sickle cell anemia in Africa: a prospective cohort study in Tanzania. *PloS one,* 2011, vol. 6, e14699
- **MCGANN et al.** A prospective newborn screening and treatment program for sickle cell anemia in Luanda, Angola. *American journal of hematology,* 2013, vol. 88, 984-989
- The Management of Sickle Cell Disease. NIH National Heart, Lung and Blood Institute, 2002
- **HERRICK J.** Peculiar elongated and sickle-shaped red blood cell corpuscles in a case of severe anemia. *Archives of Internal Medicine,* 1910
- **PAULING et al.** Sickle cell anemia, a molecular disease. *Science,* 1949, vol. 109, 443
- **NAGEL et al.** The paradox of hemoglobin SC disease. *Blood Reviews,* 2003, vol. 17, 167-178
- **PAULING et al.** *Science,* 1949, vol. 110 (2865), 543-548
- **BARABINO et al.** *Annual review of biomedical engineering,* 2010, vol. 12, 345-367
- **PLATT et al.** *N. Engl. J. Med.,* 1994, vol. 330 (23), 1639-1644
- **HILLERY et al.** *Blood,* 1996, vol. 87 (11), 4879-4886
- **KAUL et al.** *Microcirculation,* 2009, vol. 16 (1), 97-111
- **KAUL et al.** *Proceedings of the National Academy of Sciences of the United States of America,* 1989, vol. 86 (9), 3356-3360
- **HEBBEL et al.** *The New England journal of medicine,* 1980, vol. 302 (18), 992-995
- **HOOVER et al.** *Blood,* 1979, vol. 54 (4), 872-876
- **AN et al.** *British journal of haematology,* 2008, vol. 141 (3), 367-375
- **MOHANDAS.** *Blood,* 2008, vol. 112 (10), 3939-3948

- **LIPOWSKY, H. H.** *Microcirculation,* 2005, vol. 12 (1), 5-15
- **HOFRICHTER.** *Proceedings of the National Academy of Sciences of the United States of America,* 1974, vol. 71 (12), 4864-4868
- **BARABINO.** *Annual Review Of Biomedical Engineering,* 2010, vol. 12, 345-367
- **ALEXY.** *Transfusion,* 2006, vol. 46 (6), 912-918
- **HEBBEL R. P.** *Blood,* 1991, vol. 77 (2), 214-237
- **FERRONE.** *Microcirculation,* 2004, vol. 11 (2), 115-28
- **NOGUCHI.** *Annual review of biophysics and biophysical chemistry,* 1985, vol. 14, 239-263
- **NASH.** *Blood,* 1984, vol. 63 (1), 73-82
- **BRANDAO.** *European journal of haematology,* 2003, vol. 70 (4), 207-211
- **MOHANDAS.** *Journal of Clinical Investigation,* 1985, vol. 76 (4), 1605-1612
- **MONTES.** *American journal of hematology,* 2002, vol. 70 (3), 216-227
- **KASSCHAU.** *Blood,* 1996, vol. 87 (2), 771-780
- **BARTOLUCCI.** *Blood,* 2012, vol. 120 (15), 3136-3141
- **KAUL.** *Microcirculation,* 2012, vol. 16 (1), 97-111
- **KAUL.** *The Journal of clinical investigation,* 1983, vol. 72 (1), 22-31
- **LEI.** *Proceedings of the National Academy of Sciences of the United States of America,* 2013, vol. 110 (28), 11326-11330
- **BUNN.** *The New England journal of medicine,* 1997, vol. 337 (11), 762-769
- **BALLAS.** *Microcirculation,* 2004, vol. 11 (2), 209-225
- **KAUL.** *Proceedings of the National Academy of Sciences of the United States of America,* 1989, vol. 86 (9), 3356-3360
- **HEBBEL.** *Journal of Clinical Investigation,* 1997, vol. 100 (11), S83-S86
- **HEBBEL.** *The New England journal of medicine,* 1980, vol. 302 (18), 992-995

- **BALLAS.** *Blood,* 1992, vol. 79 (8), 2154-2163
- **KAUL et al.** *The Journal of clinical investigation,* 1983, vol. 72 (1), 22-31
- **FABRY et al.** *Blood,* 1982, vol. 60 (6), 1370-1377
- **KAUL et al.** *Microvascular research,* 1983, vol. 26 (2), 170-181
- **LIPOWSKY et al.** *The Journal of clinical investigation,* 1987, vol. 80 (1), 117-127
- **KAUL et al.** *Blood,* 1986, vol. 68 (5), 1162-1166
- **CONNES et al.** *British journal of haematology,* 2014, vol. 165 (4), 564-572
- **HEBBEL et al.** Erythrocyte adherence to endothelium in sickle-cell anemia. A possible determinant of disease severity. *N Engl J Med,* 1980, vol. 302, 992-995
- **HEBBEL et al.** Erythrocyte/endothelial interactions and the vasocclusive severity of sickle cell disease. *Prog Clin Biol Res,* 1981, vol. 55, 145-162
- **BARABINO et al.** Endothelial cell interactions with sickle cell, sickle trait, mechanically injured, and normal erythrocytes under controlled flow. *Blood,* 1987, vol. 70, 152-157
- **BARABINO et al.** Rheological studies of erythrocyteendothelial cell interactions in sickle cell disease. *Prog Clin Biol Res,* 1987, vol. 240, 113-127
- **SETTY et al.** Role of erythrocyte phosphatidylserine in sickle red cell endothelial Adhesion. *Blood,* 2002, vol. 99, 1564-1571
- **KAUL et al.** Sickle red cell-endothelium interactions. *Microcirculation,* 2009, vol. 16, 97-111
- **FRENETTE et al.** Sickle cell disease: old discoveries, new concepts, and future promise. *J. Clin Invest,* 2007, vol. 117, 850-858
- **BELCHER et al.** Activated monocytes in sickle cell disease: potential role in the activation of vascular endothelium and vaso-occlusion. *Blood,* 2000, vol. 96, 2451-2459
- **NATARAJAN et al.** Adhesion of sickle red blood cells and damage to interleukin-1 beta stimulated endothelial cells under flow in vitro. *Blood,* 1996, vol. 87, 4845-4852
- **PERELMAN et al.** Placenta growth factor activates monocytes and correlates with sickle cell disease severity. *Blood,* 2003, vol. 102, 1506-1514
- **ZENNADI et al.** Sickle red cells induce adhesion of lymphocytes and monocytes to endothelium. *Blood,* 2008, vol. 112, 3474-3483
- **STONE et al.** Effects of density and of dehydration of sickle cells on their adhesion to cultured endothelial cells. *Am J Hematol,* 1996, vol. 52, 135-143
- **KAUL.** Sickle erythrocyte-endothelial interactions in microcirculation: the role of von Willebrand factor and implications for vasoocclusion. *Blood,* 1993, vol. 81, 2429-2438
- **WICK.** Unusually large von Willebrand factor multimers increase adhesion of sickle erythrocytes to human endothelial cells under controlled flow. *J Clin Invest,* 1987, vol. 80, 905-910
- **BRITTAIN.** Thrombospondin from activated platelets promotes sickle erythrocyte adherence to human microvascular endothelium under physiologic flow: a potential role for platelet activation in sickle cell vaso-occlusion. *Blood,* 1993, vol. 81, 2137-2143
- **SUGIHARA.** Thrombospondin mediates adherence of CD36+ sickle reticulocytes to endothelial cells. *Blood,* 1992, vol. 80, 2634-2642
- **BARABINO.** Inhibition of sickle erythrocyte adhesion to immobilized thrombospondin by von Willebrand factor under dynamic flow conditions. *Blood,* 1997, vol. 89, 2560-2567
- **FINNEGAN.** Adherent leukocytes capture sickle erythrocytes in an in vitro flow model of vaso-occlusion. *Am J Hematol,* 2007, vol. 82, 266-275
- **TURHAN.** Primary role for adherent leukocytes in sickle cell vascular occlusion: a new paradigm. *Proc Natl Acad Sci U S A,* 2002, vol. 99, 3047-3051
- **HEBBEL.** Erythrocyte adherence to endothelium in sickle-cell anemia. *New Engl J Med,* 1980, vol. 302, 992-995
- **SOLOVEY.** Circulating activated endothelial cells in sickle cell anemia. *N Engl J Med,* 1997, vol. 337, 1584-1590
- **STRIJBOS.** Circulating endothelial cells: a potential parameter of organ damage in sickle cell anemia?. *Blood Cells Mol Dis,* 2009, vol. 43, 63-67
- **VAN BEEM.** Elevated endothelial progenitor cells during painful sickle cell crisis. *Exp Hematol,* 2009, vol. 37, 1054-1059
- **JANG.** CXCL1 and its receptor, CXCR2, mediate murine sickle cell vaso-occlusion during hemolytic transfusion reactions. *J Clin Invest,* vol. 121, 1397-1401
- **WANG et al.** Micro-a-fluidics ELISA for rapid CD4 cell count at the point-of-care. *Scientific Reports,* 2014, vol. 4, 3796
- **COLIN et al.** *Current opinion in hematology,* 2014, vol. 21 (3), 186-192
- **COOKE et al.** *Parasitology,* 1993, vol. 107, 359-368
- **TURITTO, V. T.** *Progress in hemostasis and thrombosis,* 1982, vol. 6, 139-177
- **MONTES et al.** *American journal of hematology,* 2002, vol. 70 (3), 216-227
- **KASSCHAU et al.** *Blood,* 1996, vol. 87 (2), 771-780
- **LEI et al.** *Proceedings of the National Academy of Sciences of the United States of America,* 2013, vol. 110 (28), 11326-11330

- **LORCH et al.** An elevated estimated pulmonary arterial systolic pressure, whenever measured, is associated with excess mortality in adults with sickle cell disease. *Acta Haematol,* 2011, vol. 125, 225-229
- **POWARS et al.** Outcome of sickle cell anemia: a 4-decade observational study of 1056 patients. *Medicine (Baltimore),* 2005, vol. 84, 363-376
- **NOURAIE et al.** The relationship between the severity of hemolysis, clinical manifestations and risk of death in 415 patients with sickle cell anemia in the US and Europe. *Haematologica,* 2013, vol. 98, 464-472
- **PLATT et al.** Mortality in sickle cell disease. Life expectancy and risk factors for early death. *N Engl J Med,* 1994, vol. 330, 1639-1644
- **OHENE-FREMPONG et al.** Cerebrovascular accidents in sickle cell disease: rates and risk factors. *Blood,* 1998, vol. 91, 288-294
- **WEST et al.** Laboratory profile of sickle cell disease: a cross-sectional analysis. The Cooperative Study of Sickle Cell Disease. *J Clin Epidemiol,* 1992, vol. 45, 893-909
- **CHARACHE et al.** Effect of hydroxyurea on the frequency of painful crises in sickle cell anemia. Investigators of the Multicenter Study of Hydroxyurea in Sickle Cell Anemia. *N Engl J Med,* 1995, vol. 332, 1317-1322
- **LETTRE et al.** DNA polymorphisms at the BCL11A, HBS1L-MYB, and beta-globin loci associate with fetal hemoglobin levels and pain crises in sickle cell disease. *Proc Natl Acad Sci U S A,* 2008, vol. 105, 11869-11874
- **MILTON.** Genetic determinants of haemolysis in sickle cell anaemia. *Br J Haematol,* 2013, vol. 161, 270-278
- **BAE.** Meta-analysis of 2040 sickle cell anemia patients: BCL11A and HBS1L-MYB are the major modifiers of HbF in African Americans. *Blood,* 2012, vol. 120, 1961-1962
- **MILTON.** A genome-wide association study of total bilirubin and cholelithiasis risk in sickle cell anemia. *PLoS One,* 2012, vol. 7, e34741
- **YANG.** A simple, rapid, low-cost diagnostic test for sickle cell disease. *Lab on a Chip,* 2013, vol. 13, 1464-1467
- **WOOD et al.** Differential expression of E- and P-selectin in the microvasculature of sickle cell transgenic mice. *Microcirculation,* 2004, vol. 11, 377-385
- **WOOD et al.** Endothelial cell P-selectin mediates a proinflammatory and prothrombogenic phenotype in cerebral venules of sickle cell transgenic mice. *American Journal Of Physiology Heart And Circulatory Physiology,* 2004, vol. 286, H1608-1614
- **HIDALGO et al.** Heterotypic interactions enabled by polarized neutrophil microdomains mediate thromboinflammatory injury. *Nat Med,* 2009, vol. 15, 384-391
- **ALMEIDA et al.** Hydroxyurea and a cGMP-amplifying agent have immediate benefits on acute vaso-occlusive events in sickle cell disease mice. *Blood,* 2012, vol. 120, 2879-2888
- **MATSUI et al.** P-selectin mediates the adhesion of sickle erythrocytes to the endothelium. *Blood,* 2001, vol. 98, 1955-1962
- **BELCHER et al.** *Blood,* 2000, vol. 96, 2451-2459
- **LIN et al.** Origins of circulating endothelial cells and endothelial outgrowth from blood. *J Clin Invest,* 2000, vol. 105, 71-77
- **SAMSEL et al.** Imaging flow cytometry for morphologic and phenotypic characterization of rare circulating endothelial cells. *Cytometry Part B, Clinical Cytometry,* 2013
- **MUNSON et al.** Fundamentals of Fluid Mechanics. John Wiley & Sons Canada, 2012, 792
- **WICK et al.** *Current opinion in hematology,* 1996, vol. 3 (2), 118-124
- **MOSHER, D. F.** *Annual Review of Medicine,* 1984, vol. 35, 561-575
- **KUMAR et al.** *Blood,* 1996, vol. 88 (11), 4348-4358
- Sickle-Cell Disease in the African Region: Current Situation and the Way Forward. AFR/RC56/17. World Health Organization Regional Committee for Africa, WHO, 2006
- Sickle-cell disease: a strategy for the WHO African Region: Report of the Regional Director. AFR/RC60/8. World Health Organization Regional Office for Africa, WHO, 2010
- Sickle Cell Disease and other Hemoglobin Disorders Fact Sheet. World Health Organization, 2011
- **GROSSE, S.D. ; I. ODAME ; H.K. ATRASH ; D.D. AMENDAH ; F.B. PIEL ; T.N. WILLIAMS.** Sickle cell disease in Africa: a neglected cause of early childhood mortality. *Am J Prev Med,* 2011, vol. 41 (6), S398-405
- **ODAME, I.** Perspective: We need a global solution. *Nature,* 2014, vol. 515 (7526), S10-S10
- **MAKANI, J. ; S.E. COX ; D. SOKA ; A.N. KOMBA ; J. ORUO ; H. MWAMTEMI ; P. MAGESA ; S. RWEZAULA ; E. MEDA ; J. MGAYA.** Mortality in sickle cell anemia in Africa: a prospective cohort study in Tanzania. *PLoS One,* 2011, vol. 6 (2), e14699
- **CHIODINI, P.L. ; K. BOWERS ; P. JORGENSEN ; J.W. BARNWELL ; K.K. GRADY ; J. LUCHAVEZ ; A.H. MOODY ; A. CENIZAL ; D. BELL.** The heat stability of Plasmodium lactate dehydrogenase-based and histidine-rich protein 2-based malaria rapid diagnostic tests. *Trans R Soc Trop Med Hyg,* 2007, vol. 101 (4), 331-7